# EUROPEAN PATENT SPECIFICATION

(11) **EP 2 729 810 B1**
(45) Date of publication and mention of the grant of the patent: **10.10.2018**
(21) Application number: 12740236.0
(22) Date of filing: 05.07.2012
(51) Int. Cl.: G01N 33/573, G01N 33/564

(54) **MYOSITIS**
MYOSITIS
MYOSITE

(30) Priority: 07.07.2011 US 201161505233 P; 07.07.2011 NL 2007065
(43) Date of publication of application: 14.05.2014
(73) Proprietor: Stichting Katholieke Universiteit, 6525 HP Nijmegen (NL); Stichting Katholieke Universiteit, 6525 EZ Nijmegen (NL)
(72) Inventor: PRUIJN, Gerardus Jozef Maria, NL-6642 AJ Beuningen (NL); PLUK, Wilhelmina Lamberta Leonarda Petronell, NL-5432 DR Cuijk (NL); VAN ENGELEN, Basilius Gerardus Maria, NL-6523 MT Nijmegen (NL)
(74) Representative: EP&C
(86) International application number: PCT/NL2012/050482
(87) International publication number: WO 2013/006059

(56) References cited:
- WO-A2-02/04613
- MOHAMMAD SALAJEGHEH ET AL: "Autoantibodies against a 43 KDa Muscle Protein in Inclusion Body Myositis", PLOS ONE, vol. 6, no. 5, 1 January 2011 (2011-01-01) , page E20266, XP055020569, ISSN: 1932-6203, DOI: 10.1371/journal.pone.0020266
- EL-SHAMMAA N A ET AL: "Interstitial 5'-nucleotidase stain for frozen biopsy specimens of skeletal muscle. A useful adjunct in the diagnosis of polymyositis.", ARCHIVES OF PATHOLOGY & LABORATORY MEDICINE MAR 1984 LNKD- PUBMED:6199001, vol. 108, no. 3, March 1984 (1984-03), pages 251-256, XP009156962, ISSN: 0003-9985
- HILTON D A ET AL: "Histochemical demonstration of 5'-nucleotidase activity in inflammatory muscle disease.", ARCHIVES OF PATHOLOGY & LABORATORY MEDICINE APR 1991 LNKD- PUBMED:2012497, vol. 115, no. 4, April 1991 (1991-04), pages 362-364, XP009156977, ISSN: 0003-9985
- SANDER H. J. DOOREN ET AL: "Myositis-specific autoantibodies: detection and clinical associations", AUTOIMMUNITY HIGHLIGHTS, vol. 2, no. 1, 23 March 2011 (2011-03-23), pages 5-20, XP055020327, ISSN: 2038-0305, DOI: 10.1007/s13317-011-0018-8 cited in the application
- WILSON F ABDO ET AL: "Increased plasma amyloid-Î42 protein in sporadic inclusion body myositis", ACTA NEUROPATHOLOGICA, SPRINGER, BERLIN, DE, vol. 118, no. 3, 6 June 2009 (2009-06-06), pages 429-431, XP019739819, ISSN: 1432-0533, DOI: 10.1007/S00401-009-0554-8
- J VAN DER PAS ET AL: "Diagnostic value of MHC class I staining in idiopathic inflammatory myopathies", JOURNAL OF NEUROLOGY, NEUROSURGERY, AND PSYCHIATRY, vol. 75, 1 January 2004 (2004-01-01), pages 136-139, XP055020585, DOI: http://www.ncbi.nlm.nih.gov/pmc/articles/P MC1757482/pdf/v075p00136.pdf
- WEIHL CONRAD C ET AL: "Sporadic inclusion body myositis: possible pathogenesis inferred from biomarkers", CURRENT OPINION IN NEUROLOGY, vol. 23, no. 5, October 2010 (2010-10), pages 482-488, XP009157001, ISSN: 1350-7540
- Diazyme: "5'-Nucleotidase (5'-NT)", , 8 January 2008 (2008-01-08), XP055415105, Retrieved from the Internet: URL:http://www.peramed.com/peramed/docs/DZ 123A-K.pdf [retrieved on 2017-10-12]

## Description

### FIELD OF THE INVENTION

The present invention relates to the field of myopathy and particularly to the diagnosis of inclusion body myositis (IBM). In particular the present invention envisages the use of 5'-nucleotidase in the diagnosis of a myopathy, in particular in IBM. Also provided are methods to monitor disease progression and response to treatment, methods to distinguish between different subtypes of inflammatory myopathies, as well as kits for use in the diagnosis of a myopathy, in particular inclusion body myositis.

### BACKGROUND OF THE INVENTION

Idiopathic inflammatory myopathy (IIM), also called myositis is a generic term designating autoimmune diseases of the (skeletal) muscle. Dermatomyositis (DM), Polymyositis (PM) and IBM are the major subtypes of said idiopathic inflammatory myopathies (http://www.ninds.nih.gov/disorders/inflammatory_myopathies/detail_inflammatory_myopathie s.htm; http://www.myositis.org/template/page.cfm?id=2; http://www.ncbi.nlm.nih.gov/books/NBK6196/).

DM is a muscle disease characterized by inflammation in muscle and a specific skin rash. DM most commonly occurs in children age 5 - 15 and adults age 40 - 60. Women develop the condition more often than men. Typical symptoms include difficulty swallowing, muscle weakness, stiffness, or soreness, purple or violet colored upper eyelids, purple-red skin rash and/or shortness of breath. Involvement of heart and pulmonary dysfunction are possible, in addition DM is associated with an elevated risk to develop cancer. DM is treated with corticosteroids and other drugs that suppress the immune system.

PM is a similar condition, but the symptoms can occur without a specific skin rash. It most commonly occurs between ages 50 and 70, or in children ages 5 to 15, with women twice as often affected as men. The main treatment is, again, with corticosteroid medications.

IBM is the third main subtype of the group of muscle diseases known as inflammatory myopathies. The name was first used by Yunis and Samaha in 1971 for a particular case of myopathy that phenotypically suggested the presence of chronic PM, but showed cytoplasmic vacuoles and inclusions in muscle biopsy.

The onset of muscle weakness in IBM is generally gradual (over months or years) and affects both proximal (close to the trunk of the body) and distal (further away from the trunk) muscles. For some individuals, the disorder begins with weakness in the wrists and fingers that causes difficulty with pinching, buttoning, and gripping objects. Also difficulty swallowing
occurs in approximately half of IBM cases. Patients may eventually end up in a wheelchair and at later stages of the disease the majority of the patients require major assistance for their daily lives.

IBM is the most commonly acquired muscle disease in people over 45, although the disease can occur earlier. IBM occurs more frequently in men than in women and is characterized by a combination of muscle degeneration and inflammation (See Needham M, Neuromuscul Disord. 2008;18:6-16 and Amato AA, Journal of neurology, neurosurgery, and psychiatry. 2009;80:1186-1193). In contrast to DM and PM, IBM is generally resistant to all therapies and its rate of progression appears to be unaffected by currently available treatments.

WO2006115448 suggests the use of growth hormone, a secretagogue thereof or a mixture thereof, to cure IBM or suppress the symptoms associated therewith.

Circulating autoantibodies have been detected in DM and PM (see for reviews Mammen AL (2010) Ann N Y Acad Sci 1184: 134-153 and Van Dooren et al. (2011) Autoimmun Highlights 2: 5-20). These antibodies, for example directed against aminoacyl-tRNA synthetases, occur with differing prevalences. Myositis-specific autoantibodies (MSA) such as anti-Jo-1 and anti-Mi-2 are associated with specific clinical phenotypes in PM and DM but are only rarely found in IBM. This knowledge is used in the diagnosis of these conditions.

Currently, diagnosing IBM is dependent on an invasive diagnostic procedure which includes examining a patients' muscle biopsy or repeated muscle biopsies for the presence of rimmed vacuoles and protein inclusions. Due to selection bias, vacuoles can be missed in the biopsied muscle tissue, in which case repeated biopsies are necessary to establish the diagnosis. Unfortunately misdiagnosis due to erroneous interpretation of biopsies is a problem in differentiating PM and/or DM from IBM. Moreover, a muscle biopsy is an invasive procedure. This emphasizes the need for novel, more specific and more patient friendly methods in the diagnosis of inflammatory myopathy, in particular for diagnosing IBM, and distinguishing it from PM and/or DM. Targeting the correct subtype in a human subject will prevent the unnecessary application of potentially toxic therapies.

### SUMMARY OF THE INVENTION

The invention is as described in the claims.

The present inventors have now identified the antigen 5-nucleotidase and fragments thereof as an autoantibody target in IBM. Additionally, the present inventors have identified regions of 5'-nucleotidase that may function as such autoantibody target and may comprise one or more epitopes for the autoantibody.
Thus, in a first aspect, the present invention relates to a method for identifying a subject at risk of developing Inclusion Body Myositis and/or diagnosing a subject suffering from Inclusion Body Myositis, said method comprising the steps of:
a) providing a test sample of said subject;
b) determining whether antibodies against 5'-nucleotidase are present in said sample.

Both the level or the mere presence may be determined in the methods according to the invention. The presence of said antibodies against 5'-nucleotidase and/or a fragment thereof is indicative of a risk of developing or having the idiopathic inflammatory myopathy Inclusion Body Myositis. At the same time the presence of said antibodies against 5'-nucleotidase and/or a fragment thereof is also indicative against a risk of developing or having the idiopathic inflammatory myopathy dermatomyositis and/or polymyositis. Thus, the present invention also provides a method for distinguishing between subtypes of idiopathic inflammatory myopathy.

In a second aspect, the invention provides a method for monitoring progression of Inclusion Body Myositis in a subject and/or for determining response to therapy addressing Inclusion Body Myositis in a subject, said method comprising the steps of:
a) providing a first test sample of said subject at a first time point, and a second test sample of said subject at a second time point;
b) determining the level of antibodies against 5'-nucleotidase in said first and second samples;
c) comparing the level of antibodies against 5'-nucleotidase in said first sample to said second sample.

The method may include (d) determining progression of Inclusion Body Myositis in said subject and/or the response to therapy addressing Inclusion Body Myositis based upon the comparison of the level of antibodies against 5'-nucleotidase, or fragments thereof between said first sample and said second sample. Progression of Inclusion Body Myositis in said subject and/or the response to therapy addressing Inclusion Body Myositis is based upon the comparison of the level of antibodies against 5'-nucleotidase and fragments between said first sample and said second sample.

The method according to the invention is preferably a method wherein the 5'-nucleotidase is NT5C1A (human 5'-nucleotidase, cytosolic IA) or NT5C1B (human 5'-nucleotidase, cytosolic IB), preferably NT5C1A, or a fragment obtained therefrom.

Alternatively, the method according to the invention is preferably a method wherein the 5'-nucleotidase comprises at least 30, 40, 50, 100, 200, 300, preferably at least 350, most preferably all adjacent amino acids of SEQ ID NO: 1 and/or SEQ ID NO: 2, or of an amino acid sequence having at least 90%, more preferably at least 95%, even more preferably at least 98%, most preferably at least 99% identity with SEQ ID NO:1 and/or SEQ ID NO:2. For example, advantageously the 5'-nucleotidase may comprise one or more of the regions that may function as an autoantibody target and may comprise one or more epitopes for the autoantibody set forth herein. Non-limiting examples of such regions include the region of amino acids 25-50, 221-243, and 341-368 of 5'-nucleotidase IA.

In a preferred aspect of the invention there is provided a method wherein the actual step of determining the presence and/or level of antibodies against 5'-nucleotidase, is performed by determining interaction, binding, association between said antibodies with 5'-nucleotidase and/or with a fragment of said 5'-nucleotidase, wherein said fragment is a fragment of at least 7 adjacent amino acids derived from said 5'-nucleotidase. The 5'-nucleotidase, or the fragment, may be from mammalian origin, be synthetic, or be produced by any means, as long as it is able to (specifically) recognize and bind with (auto)antibodies that may be present in a sample obtained from a patient with Inclusion Body Myositis, which (auto)antibodies bind to human 5'-nucleotidase, preferably the NT5C1A or NT5C1B protein. In general the determination of the presence of antibodies in a sample, as disclosed herein is preferably performed ex vivo, when the subject is a human or animal, in vitro, or ex situ.

In a preferred embodiment the 5'-nucleotidase used to determine interaction with the antibody, or from which the fragment used to determine interaction with the antibody is derived, is NT5C1A (human 5'-nucleotidase, cytosolic IA) or NT5C1B (human 5'-nucleotidase, cytosolic IB). In another preferred embodiment the 5'-nucleotidase used to determine interaction with the antibody, or from which the fragment used to determine interaction with the antibody is derived, comprises at least 30, 40, 50, 100, 200, 300, preferably at least 350, most preferably all adjacent amino acids of SEQ ID NO: 1 and/or SEQ ID NO: 2, or of an amino acid sequence having at least 90%, more preferably at least 95%, even more preferably at least 98%, most preferably at least 99% identity with SEQ ID NO:1 and/or SEQ ID NO:2. In an embodiment, the 5'-nucleotidase used to determine interaction with the antibody encompasses one or more of the regions that may function as an autoantibody target and may comprise one or more epitopes for the autoantibody set forth herein. Non-limiting examples of such regions include the region of amino acids 25-50, 221-243, and 341-368 of 5'-nucleotidase IA.

The test sample used in the methods according to the invention is preferably a blood sample, which blood sample may, before determining the presence of (auto)antibodies against the 5'-nucleotidase, be further treated or purified. The subject is preferably a human subject.

Also provided is that methods of the current invention further comprise the step of determining the presence of antibodies against at least one antigen selected from the group consisting of Mi-2, Ku, PM/Scl-100, PM/Scl-75, SRP, OJ, EJ, PL-12, PL-7, Ro-52, Jo-1, HisRS, ThrRS, AlaRS, GlyRS, IleRS, AsnRS, TyrRS, PheRS, tRNA^{His}, tRNA^{Ala}, Mi-2alpha, Mi-2beta, SRP54, SRP68, SRP72, Tif1-gamma, MDA5, SAE1, SAE2, serine-tRNA^{Sec}-protein complex, Ro60, La, U1A, U1C, U1-70k, PMS1, PMS2, Ku70, Ku80, eEF1, nuclear RNP and NXP-2 in the test sample.

The antigens above comprise targets of autoantibodies regularly found in IIM patients. The latter include myositis associated autoantibodies (MAA), which are not specific and are also found in other rheumatic disorders, and myositis-specific autoantibodies (MSA),which are found primarily in patients with IIM (See Van Dooren et al. (2011) Autoimmun Highlights 2: 5-20). By determining the presence or absence of antibodies against these additional antigens, and/or fragments thereof, diagnosis of a subject is further improved.

In a further aspect, the present invention pertains to the use of 5'-nucleotidase and/or use of a fragment of said 5'-nucleotidase, wherein said fragment is a fragment of at least 7, such as 8, 9, 10, 11, 12, 13, 14,15 or more adjacent amino acids derived from said 5'-nucleotidase, preferably from the regions referred to above, for detecting antibodies present in a test sample obtained from a subject. In an embodiment, the 5'-nucleotidase, or the fragment, may be from mammalian origin, be synthetic, or be produced by any means, as long as it is able to (specifically) recognize and bind with (auto)antibodies that may be present in a sample obtained from a patient with Inclusion Body Myositis, which (auto)antibodies bind to human 5'-nucleotidase, preferably the NT5C1A or NT5C1B protein. In general the determination of the presence of antibodies in a sample, as disclosed herein is preferably performed ex vivo, when the subject is a human or animal, in vitro, or ex situ. In other words, the fragments of at least 5, preferably at least 6, more preferably at least 7 adjacent amino acids derived from said 5'-nucleotidase, are such that they can be used to bind or interact with the (auto)antibodies present in a sample obtained from a subject with Inclusion Body Myositis, and that normally would bind with the 5'-nucleotidase, thereby detecting the presence of such antibodies in a sample and allowing the diagnosis of Inclusion Body Myositis.

The 5'-nucleotidase, or the fragment derived therefrom, used to determine interaction with an antibody present in a sample is preferably NT5C1A (human 5'-nucleotidase, cytosolic IA) or NT5C1B (human 5'-nucleotidase, cytosolic IB).

Alternatively, the 5'-nucleotidase used to detect the antibodies, or from which the fragment used to determine interaction with the antibody is derived, comprises at least 30, 40, 50, 100, 200, 300, preferably at least 350, most preferably all adjacent amino acids of SEQ ID NO: 1 and/or SEQ ID NO: 2, or of an amino acid sequence having at least 90%, more preferably at least 95%, even more preferably at least 98%, most preferably at least 99% identity with SEQ ID NO:1 and/or SEQ ID NO:2.

The use according to the above aspect is in particular useful in identifying a subject at risk of developing Inclusion Body Myositis and/or diagnosing a subject suffering from Inclusion Body Myositis, and/or monitoring progression of Inclusion Body Myositis in a subject.

In a preferred embodiment the test sample is a blood sample. In a preferred embodiment the subject is a human subject.

In a final aspect, the present invention relates to a kit according to claim 8. The application provides a kit comprising means for detecting antibodies against 5'-nucleotidase present in a test sample taken from a subject, preferably wherein the 5'-nucleotidase is NT5C1A (human 5'-nucleotidase, cytosolic IA) or NT5C1B (human 5'-nucleotidase, cytosolic IB) or a 5'-nucleotidase comprising at least 30, 40, 50, 100, 200, 300, preferably at least 350, most preferably all adjacent amino acids of SEQ ID NO: 1 and/or SEQ ID NO: 2, or an amino acid sequence having at least 90%, more preferably at least 95%, even more preferably at least 98%, most preferably at least 99% identity with SEQ ID NO:1 and/or SEQ ID NO:2.

The kit according to the invention is particular useful in identifying a subject at risk of developing Inclusion Body Myositis and/or diagnosing a subject suffering from Inclusion Body Myositis, and/or in monitoring progression of Inclusion Body Myositis in a subject.

In an embodiment the kit comprises 5'-nucleotidase, preferably wherein the 5'-nucleotidase is NT5C1A (human 5'-nucleotidase, cytosolic IA) or NT5C1B (human 5'-nucleotidase, cytosolic IB) or a 5'-nucleotidase comprising at least 30, 40, 50, 100, 200, 300, preferably at least 350, most preferably all adjacent amino acids of SEQ ID NO: 1 and/or SEQ ID NO: 2, or an amino acid sequence having at least 90%, more preferably at least 95%, even more preferably at least 98%, most preferably at least 99% identity with SEQ ID NO:1 and/or SEQ ID NO:2, or a fragment of said 5'-nucleotidase or said amino acid sequence, wherein said fragment is a fragment of at least 5, preferably at least 6, more preferably at least 7 adjacent amino acids derived from said 5'-nucleotidase or said amino acid sequence, as means for detecting the anti-bodies against the 5'-nucleotidase.

The kit according to the invention is a kit wherein said 5'-nucleotidase and/or fragment to detect antibodies against 5'-nucleotidase is affixed to a membrane suitable for use in *in vitro* immunoassays, preferably an immunoblot, beads, magnetic beads, carriers, protein-linkers.

In one embodiment the kit further comprises an assay for the detection of binding of antibodies against 5'-nucleotidase, preferably the assay is selected from the group consisting of a molecular interaction assay, ELISA, immunoblotting, microarrays, immunoprecipitation, immunodiffusion, counterimmunoelectrophoresis, or multiplexed analysis techniques.

The kit of the current invention may advantageously further comprise means for detecting antibodies against at least one antigen selected from the group consisting of Mi-2, Ku, PM/Scl-100, PM/Scl-75, SRP, OJ, EJ, PL-12, PL-7, Ro-52, Jo-1, HisRS, ThrRS, AlaRS, GlyRS, IleRS, AsnRS, TyrRS, PheRS, tRNA^{His}, tRNA^{Ala}, Mi-2alpha, Mi-2beta, SRP54, SRP68, SRP72, Tif1-gamma, MDA5, SAE1, SAE2, serine-tRNA^{Sec}-protein complex, Ro60, La, U1A, U1C, U1-70k, PMS1, PMS2, Ku70, Ku80, eEF1, nuclear RNP and NXP-2. For example, the kit may comprise these antigens, of fragments thereof, in the same manner as described above for 5'-nucleotidase. The combination of these means, e.g. antigens, or fragments thereof, in a kit together with the above described means to detect the presence of (auto)antibodies against 5'-nucleotidase allows for the detection of a panel of antibodies present in a sample. In case the sample is derived from a subject, this allows for an improved diagnosis of the subject, and significantly reduces the change of misdiagnosis or misinterpretation of the data obtained.

### GENERAL DEFINITIONS

In the following description and examples, a number of terms are used. In order to provide a clear and consistent understanding of the specification and claims, including the scope to be given such terms, the following definitions are provided. Unless otherwise defined herein, all technical and scientific terms used have the same meaning as commonly understood by one of ordinary skill in the art to which this invention belongs.

In this document and in its claims, the verb "to comprise" and its conjugations is used in its non-limiting sense to mean that items following the word are included, but items not specifically mentioned are not excluded. The term also encompasses "to consist essentially of" and "to consist of". In addition, reference to an element by the indefinite article "a" or "an" does not exclude the possibility that more than one of the element is present, unless the context clearly requires that there be one and only one of the elements. The indefinite article "a" or "an" thus usually means "at least one". It is further understood that, when referring to "sequences" herein, generally the actual physical molecules with a certain sequence of subunits (e.g. amino acids) are referred to.

The term "and/or" indicates indicate that one or more of the stated cases may occur. In other words, a stated case may either occur alone or in combination with at least one of the stated cases, up to with all of the stated cases. The term and/or discloses each stated case have similar lengths. Generally, the GAP default parameters are used, with a gap creation penalty = 50 (nucleotides) / 8 (proteins) and gap extension penalty = 3 (nucleotides) / 2 alone, as well as the specific combination of a stated case with at least one of the other stated cases, up to with all of the stated cases.

The term "antibody" used herein refers to any immunoglobulin or fragment thereof, and encompasses any polypeptide comprising an antigen-binding site with at least one complementarity determining region (CDR). The term includes, but is not limited to, polyclonal, monoclonal, monospecific, polyspecific, non-specific, humanized, chimeric, human, single-chain, synthetic, recombinant, hybrid, mutated, grafted and *in vitro* generated antibodies. The term "antibody" also includes antibody fragments such Fab, F(ab')₂, Fv, scFv, Fd, dAb, and other antibody fragments or other constructs comprising CDRs that retain antigen-binding function. Typically, such fragments would comprise an antigen-binding domain. The details of the preparation of such antibodies and their suitability for use as binding members, particularly a specific binding member, are well known to those skilled in the art.

The antibody or fragment thereof may be any of the known antibody isotypes and their conformations, for example, IgA, such as IgA1 or IgA2, IgD, IgE, IgG, such as IgG1, IgG2a, IgG2b, IgG3, IgG4, or IgM class, or may constitute mixtures thereof in any combination, such as a mixture of antibodies from the IgG1 and IgG2a class.

The term "synthetic" designates non-naturally occurring material, produced by genetic engineering, synthesis, computerization, library screening, etc. in vitro, ex vivo or in vivo. Synthetic also includes artificial material, representing all or part of naturally-occurring material, optionally comprising modified structure(s) or moiety(ies).

Gene means any coding nucleic acid molecule. The term "gene" includes not only genomic DNA, but also cDNA, synthetic DNA, RNA, etc.

The term protein is used interchangeably with "polypeptide" and designates any molecule comprising an amino acid or an amino acid chain, optionally modified, glycosylated, etc.

The term antigen (or antigenic molecule) designates any molecule such as a protein, polypeptide, peptide, lipid, nucleic acid, polysaccharide, epitope, etc. against which an immune response is sought or obtained, or a nucleic acid encoding the same.

A fragment with the context of the current invention is a part of at least 7 adjacent amino acids (or a nucleic acid encoding these sequences of amino acids), derived from or present in a protein (adjacent, next to each other, in the same sequence), and that can act as an antigen, i.e. can be recognized by an antibody that would also recognize the protein, comprising, adjacently, the at least 7 amino acids. Such fragment may comprise at least part of, but preferably the epitope, also known as antigenic determinant, i.e. the part of the complete protein antigen that is recognized by the immune system/paratope of the antibody. For example, in case an antigen comprises 100 amino acids, and amino acids 50 - 58 form the epitope recognized by the antibody, a fragment of the antigen preferably is a fragment comprising the amino acid sequence 50 - 58. It will be understood by the skilled person that the amino acid fragment may be flanked on the C-terminus and/or on the N-terminus by other compounds, for example other amino acids, even if such amino acids would not flank the fragment in the antigen from which the fragment is obtained.

A nucleotidase is a hydrolytic enzyme that catalyses the hydrolysis of a nucleotide into a nucleoside and a phosphate. For example, they convert adenosine monophosphate to adenosine, and guanosine monophosphate to guanosine. 5' nucleotidases cleave off the phosphate from the 5' end of the sugar moiety of nucleic acids. They are classified into various kinds depending on their substrate preferences and subcellular localization. Membrane bound 5' nucleotidases display specificity towards adenosine monophosphates and are predominantly involved in the salvage of preformed nucleotides and in signal transduction cascades involving purinergic receptors. Soluble 5' nucleotidases are all known to belong to the haloacid dehalogenase superfamily of enzymes. One example is NT5C1A, 5'-nucleotidase, cytosolic IA (HGNC:17819), sometimes also referred to as AMP-specific 5'-NT", CN-I, CN-IA, CN1, CN1A, "cytosolic 5' nucleotidase, type 1A", "cytosolic 5'-nucleotidase IA", MGC119199, or MGC119201, which is encoded by the gene with GenBank ID: AF331801.1, and is a protein of 368 amino acids. It is highly expressed in skeletal muscle and detected at intermediate levels in heart, brain, kidney and pancreas. It belongs to the 5'-nucleotidase type 3 family. Another example is NT5C1B, 5'-nucleotidase, cytosolic IB (HGNC:17818), sometimes also referred to as AIRP, autoimmune infertility-related protein, Cytosolic 5'-nucleotidase IB or CN-IB, is encoded by the gene with GenBank ID: AF356185.1, and is a protein of 610 AA. It is highly expressed in testis, placenta and pancreas. It is detected at lower levels in heart, kidney, liver and lung. See http://www.genenames.org/data/hgnc_data.php?match=NT5C1A or http://www.genenames.org/data/hgnc_data.php?match=NT5C1B for further details.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithms (e.g. Needleman Wunsch) which aligns the sequences optimally over the entire length, while sequences of substantially different lengths are preferably aligned using a local alignment algorithm (e.g. Smith Waterman). Sequences may then be referred to as "substantially identical" or "essentially similar" when they (when optimally aligned by for example the programs GAP or BESTFIT using default parameters) share at least a certain minimal percentage of sequence identity (as defined below). GAP uses the Needleman and Wunsch global alignment algorithm to align two sequences over their entire length (full length), maximizing the number of matches and minimizing the number of gaps. A global alignment is suitably used to determine sequence identity when the two sequences (proteins). For nucleotides the default scoring matrix used is nwsgapdna and for proteins the default scoring matrix is Blosum62 (Henikoff & Henikoff, 1992, PNAS 89, 915-919). Sequence alignments and scores for percentage sequence identity may be determined using computer programs, such as the GCG Wisconsin Package, Version 10.3, available from Accelrys Inc., 9685 Scranton Road, San Diego, CA 92121-3752 USA, or using open source software, such as the program "needle" (using the global Needleman Wunsch algorithm) or "water" (using the local Smith Waterman algorithm) in EmbossWIN version 2.10.0, using the same parameters as for GAP above, or using the default settings (both for 'needle' and for 'water' and both for protein and for DNA alignments, the default Gap opening penalty is 10.0 and the default gap extension penalty is 0.5; default scoring matrices are Blossum62 for proteins and DNAFull for DNA). When sequences have a substantially different overall lengths, local alignments, such as using the Smith Waterman algorithm, are preferred. Alternatively percentage similarity or identity may be determined by searching against public databases, using algorithms such as FASTA, BLAST, etc.

### SEQUENCES REFFERED TO

SEQ ID NO:1: Amino acid sequence of NT5C1A
SEQ ID NO:2: Amino acid sequence of NT5C1B

### DETAILED DESCRIPTION OF THE INVENTION

The present inventors have now identified 5'-nucleotidase as a target molecule of (auto)antibodies in IBM. IBM is histopathologically characterized by both degenerative and autoimmune features and the inventors have now recognized a skeletal muscle antigen of 44 kDa as an autoantibody target in IBM. Reactivity with the antigen in DM and PM was 0% and 2%, respectively. The antigen was identified as a 5'-nucleotidase (see below) and can be used in the detection of antibodies that are present in samples obtained from subjects with IBM. In addition, also the novel IBM-specific autoantibodies may be used as biomarker to facilitate the diagnosis of IBM. For example, purified (recombinant) 5' nucleotidase or synthetic peptides derived from this protein can now advantageously be used in biomolecular interaction assays in a diagnostic test for the detection of anti-5'-nucleotidase antibodies.

WO0204613 discloses methods to detect 5'-nucleotidases. Diazyme discioses a kit for the determination of "5'-nucleotidase (5'-NT)" activity in human serum samples (Diazyme: "5'-Nucleotidase (5-NI)", 2008).

Currently, several serological tests for myositis are commercially available, but all of these lack IBM-specific markers. Examples are the Euroline myositis blot assay and the Myositis plus test developed by Euroimmun (http://www.euroimmun.com/) and Orgentec (http://www.orgentec.com/), respectively. The implementation of 5'-nucleotidase as a IBM-specific marker in these tests would now allow for the easy and straightforward diagnosis of IBM. Also an ELISA test using 5'-nucleotidase (or peptides derived from its sequence) can be easily developed and standardized.

Therefore, in a first aspect a method is provided for identifying a subject at risk of developing an idiopathic inflammatory myopathy and/or diagnosing a subject suffering from an idiopathic inflammatory myopathy, preferably wherein said idiopathic inflammatory myopathy is Inclusion Body Myositis, said method comprising the steps of:
a) providing a test sample of said subject;
b) determining whether antibodies against 5'-nucleotidase are present in said sample.

It has now surprisingly been found by the current inventors that subjects suffering from, or at risk of developing, Inclusion Body Myositis can for the first time be diagnosed by analyzing the presence of (auto)antibodies against 5'-nucleotidase in a sample obtained from such subject. At the same time it was found that these (auto)antibodies are virtually absent in samples obtained from subjects suffering from, or at risk of developing, other idiopathic inflammatory myopathies, including Dermatomyositis and/or Polymyositis. In other words, the current invention can be both used to assess the risk of developing or having the idiopathic inflammatory myopathy Inclusion Body Myositis as well as to assess the risk of developing or having another idiopathic inflammatory myopathy like Dermatomyositis and/or Polymyositis.

In an aspect the invention pertains to a method for distinguishing between subtypes of IIM, said method comprising the step of a) providing a test sample of said subject; and b) determining whether antibodies against 5'-nucleotidase are present in said sample. The presence of said antibodies against 5'-nucleotidase is indicative of a risk of developing or having the idiopathic inflammatory myopathy Inclusion Body Myositis, and the presence of said antibodies against 5'-nucleotidase is indicative against a risk of developing or having an idiopathic inflammatory myopathy like Dermatomyositis and/or Polymyositis. I.e., when the test sample comprises antibodies against 5'-nucleotidase, a subject may be considered at risk of developing IBM or may be suffering from IBM. At the same time, it points away from the subject being at risk of developing DM and/or PM or of suffering from DM and/or PM.

Alternatively, the level of antibodies against 5'-nucleotidase in a test sample may be compared to a reference sample, for example of a healthy subject not diagnosed with an idiopathic inflammatory myopathy, e.g. not diagnosed with Inclusion Body Myositis. An increased level of said antibody in said sample compared to the reference level may be indicative of a risk of developing, or suffering from Inclusion Body Myositis. A decreased, similar or identical level of said antibody in said sample compared to the reference level may be indicative of no or a low risk of developing or suffering from Inclusion Body Myositis.

The method according to the invention may also be utilized for the immediate or early onset detection of a myopathy, preferably Inclusion Body Myositis.

Preferably the method(s) according to the invention is/are carried out ex *vivo,* i.e., on an ex *vivo* test sample. Said test sample may be any sample obtained from a subject, and is not limited to tissue or fluid obtained from a subject. A variety of samples can be useful in practicing the invention including, for example, blood, serum, plasma, urine, salivary fluid, ascites fluid, and the like. Preferably the test sample is a blood sample, non-limiting examples of such sample is a whole blood sample, but also include blood samples subsequently treated (e.g. fractionated). The skilled person is well aware of techniques and method to obtain a test sample of a subject.

In the context of the invention, a subject may be an animal or a human being. In principle, any subject could be diagnosed using the method of the invention. The diagnosis method may be applied as often as necessary for that subject. Preferably, the subject is a human being, preferably a human being of 40 years or older. In a suitable embodiment, the subject is a subject suspected to be at risk of developing an idiopathic inflammatory myopathy and/or suspected to be suffering from an idiopathic inflammatory myopathy, preferably wherein said idiopathic inflammatory myopathy is Inclusion Body Myositis. A subject may be suspected to be at risk of developing an idiopathic inflammatory myopathy based on the occurrence of symptoms regularly associated with idiopathic inflammatory myopathies, and as described above.

The determination of the presence or level of antibodies against 5'-nucleotidase in a test sample can be performed by any suitable methodology available to the skilled person, e.g. by using an immunoassay, like ELISA, immunoprecipitation or cell screening, or even by using anti-antibodies (i.e. antibodies against the antibodies against 5'-nucleotidase).

Utilizing current antibody detection techniques that can quantitate the binding of antibodies to antibodies antigens one can determine the level or amount of said antibodies in a sample obtained from a subject.

Antibodies can be used as specific analytic reagents to quantify the amount of a protein or other antigen. This technique is the enzyme-linked immunosorbent assay (ELISA). In this method, an enzyme, which reacts with a colorless substrate to produce a colored product, is covalently linked to a specific antibody that recognizes a target antigen. If the antigen is present in a sample, the antibody-enzyme complex will bind to it, and the enzyme component of the antibody-enzyme complex will catalyze the reaction generating the colored product. Thus, the presence of the colored product indicates the presence of the antigen. Such an ELISA, which is rapid and convenient, can detect less than a nanogram (10⁻⁹ g) of a protein. ELISA can be performed with either polyclonal or monoclonal antibodies, but the use of monoclonal antibodies yields more reliable results.

In case of detecting antibodies in a sample, as in the current invention, an indirect ELISA can be used. The indirect ELISA is used to detect the presence of antibodies via antigens that are absorbed to a solid support, for example the bottom of a well. A sample obtained from a subject and which may comprise antibodies against the antigen is added to the antigen-coated well and allowed to bind to the antigen. Finally, enzyme-linked antibodies to human antibodies (for instance, goat antibodies that recognize human antibodies) are allowed to react in the well and unbound antibodies are removed by washing. Substrate is then applied. The conversion of substrate indicates that the enzyme-linked antibodies were bound to human antibodies, which in turn implies that the patient had antibodies to the antigen. Thus in the case of fluorescence ELISA, when light of the appropriate wavelength is shone upon the sample, any antigen/antibody complexes will produce fluorescence so that the amount of antibody in the sample can be inferred through the magnitude of the fluorescence signal.

The substrates may utilize chromogenic substrates, though fluorogenic substrates and chemoluminescent substrates are used more commonly as they enable higher sensitivity.

Another antibody detection method is immunoprecipitation (IP). During an IP experiment, a test sample, e.g. a blood sample, is exposed to a specific antigen. If the antibody being tested is present, it will bind to the antigen, and all other antibodies will remain unbound. If the specific antibody is not present, none of the antibodies will bind to the antigen. After allowing time for antibody-antigen binding, all the antibodies, along with any antigen bound to the antibodies, are removed from the sample and analyzed.

Cell screening makes use of cells that contain specific antigens. The screening cell population is incubated with a test sample, e.g. a patient's serum sample, and antibodies from the sample are allowed to bind to antigens on the screening cells. Researchers can then detect antibody-antigen binding by analyzing the screening cells for the presence of antibodies. If the researchers find antibody-antigen complexes in the screening cells, this indicates the presence of the antibody within the patient sample.

Alternatively, the amount or presence of the antibodies in the sample of a subject may be determined using any other routine techniques known to the skilled person, including, but not limited to, capillary action, precipitation, turbidimetric, diffusion, agglutination, potentiometric, amperometric, piezoelectric and evanescent-wave immunosensors, or any combination of the methods recited herein.

Also provided is a method for monitoring progression of Inclusion Body Myositis in a subject and/or for determining response to therapy addressing Inclusion Body Myositis in a subject, said method comprising the steps of:
a) providing a first test sample of said subject at a first time point, and a second test sample of said subject at a second time point;
b) determining the level of antibodies against 5'-nucleotidase in said first and second samples;
c) comparing the level of antibodies against 5'-nucleotidase in said first sample to said second sample.

Also provided is the said method wherein the method comprises d) determining progression of Inclusion Body Myositis in said subject and/or the response to therapy addressing Inclusion Body Myositis based upon the comparison of the level of antibodies against 5'-nucleotidase between said first sample and said second sample.

Preferably the method(s) according to the invention is/are carried out ex *vivo,* i.e., on an ex *vivo* test sample. Said test sample may be any sample obtained from a subject, and is not limited to tissue or fluid obtained from a subject. A variety of samples can be useful in practicing the invention including, for example, blood, serum, plasma, urine, salivary fluid, ascites fluid, and the like. Preferably the test sample is a blood sample, non-limiting examples of such sample is a whole blood sample, but also include blood samples subsequently treated (e.g. fractionated).

This method comprises the steps of providing a first sample from said subject at a first time point, a second sample from said subject at a second time point, and determining the level of antibodies against 5'-nucleotidase in both samples. By comparing these, progression of Inclusion Body Myositis in said subject may be determined. By measuring the level of antibodies in a subject sample over time, a clinician will be able to determine whether the Inclusion Body Myositis has progressed and/or whether, for example, treatment was successful or useful.

The term "therapy addressing Inclusion Body Myositis" as used herein refers to any treatment of any kind expected to ameliorate or reverse Inclusion Body Myositis in a subject. The subject may be a positive responder, poor responder, or non-responder. For use herein, a positive responder is a subject who positively responds to treatment, i.e., a subject who experiences success in amelioration of Inclusion Body Myositis. A non-responder is a subject who does not respond to the treatment or does not respond to a satisfactory level. A poor responder is a subject who responds to treatment but not at the level of the positive responder.

A similar or identical level of antibodies against 5'-nucleotidase in said first and second samples may be indicative of a moderate response to therapy or with arrest of progression of Inclusion Body Myositis. If said second sample is taken at a later time point than said first sample, an increased level of said antibodies against 5'-nucleotidase in said second sample compared to the level of said biomarker in said first sample may be indicative of negative or low response to therapy, or to progression of Inclusion Body Myositis (non-responder). If said second sample is taken at a later time point than said first sample, a decreased level of said antibody against 5'-nucleotidase in said second sample compared to the level of said antibody against 5'-nucleotidase in said first sample may be indicative of positive response to therapy (positive responder).

In a preferred embodiment of the methods, uses and kits of the invention the 5'-nucleotidase against which the antibodies to be detected are directed is NT5C1A (human 5'-nucleotidase, cytosolic IA) or NT5C1B (human 5'-nucleotidase, cytosolic IB), preferably NT5C1A. A skilled person will understand that the antibodies, as for example present in a test sample obtained from a subject suffering from Inclusion Body Myositis, may, in addition to recognizing the 5'-nucleotidase, preferably NT5C1A and/or NT5C1B, recognize certain fragments or parts of said antigens.

Alternatively, in the methods, uses and kits of the invention, the 5'-nucleotidase against which the antibodies to be detected comprises at least 30, 40, 50,100, 200, 300, preferably at least 350, most preferably all adjacent amino acids of SEQ ID NO: 1 and/or SEQ ID NO: 2, or of an amino acid sequence having at least 90%, more preferably at least 95%, even more preferably at least 98%, most preferably at least 99% identity with SEQ ID NO:1 and/or SEQ ID NO:2. It will be understood by the skilled person that it is not necessary for the current invention that the 5'-nucleotidase according to the current invention and to which the antibodies in a subject suffering from Inclusion Body Myositis are directed has an amino acid sequence fully identical to the amino acid sequence of SEQ ID NO:1 and or SEQ ID NO:2, but that variations in the amino acid sequence (e.g. isoforms or homologs) are allowed without deviating from the invention. Likewise, the current invention does include the embodiment that only fragments of the 5'-nucleotidase as described above, and against which the antibodies in patients suffering from Inclusion Body Myositis are directed, for example fragments of at least 30, 40, 50,100, 200, 300, preferably at least 350 adjacent amino acids, are sufficient to detect an antibody response in said patients.

In an embodiment, such fragments comprises at least one amino acid region of 5'-nucleotidase IA selected from the group consisting of:
- a region of amino acid 25 to 50 of SEQ ID NO:1 or an amino acid sequence having at least 90%, more preferably at least 95%, even more preferably at least 98%, most preferably at least 99% identity with SEQ ID NO:1;
- a region of amino acid 221 to 243 of SEQ ID NO:1 or an amino acid sequence having at least 90%, more preferably at least 95%, even more preferably at least 98%, most preferably at least 99% identity with SEQ ID NO:1; and
- a region of amino acid 341 to 368 of SEQ ID NO:1 or an amino acid sequence having at least 90%, more preferably at least 95%, even more preferably at least 98%, most preferably at least 99% identity with SEQ ID NO:1.

It was found that these regions were preferentially targeted by IBM sera. These regions may represent major epitope regions.

In a particularly preferred embodiment of the current invention the step of determining the presence and/or level of antibodies against 5'-nucleotidase is performed by determining interaction between said antibodies and 5'-nucleotidase and/or with a fragment of said 5'-nucleotidase, wherein said fragment is a fragment of at least 5, preferably at least 6, more preferably at least 7 adjacent amino acids derived from said 5'-nucleotidase. Preferably the 5'-nucleotidase used to determine interaction with the antibody, or from which the fragment used to determine interaction with the antibody is derived, is NT5C1A (human 5'-nucleotidase, cytosolic IA) or NT5C1B (human 5'-nucleotidase, cytosolic IB). Alternatively the 5'-nucleotidase used to determine interaction with the antibody, or from which the fragment used to determine interaction with the antibody is derived, comprises at least 30, 40, 50, 100, 200, 300, preferably at least 350, most preferably all adjacent amino acids of SEQ ID NO: 1 and/or SEQ ID NO: 2, or of an amino acid sequence having at least 90%, more preferably at least 95%, even more preferably at least 98%, most preferably at least 99% identity with SEQ ID NO:1 and/or SEQ ID NO:2.

In an embodiment, the fragment comprises at least 5, preferably at least 6, more preferably at least 7 adjacent amino acids from a region of 5'-nucleotidase selected from the group consisting of: - a region of amino acid 25 to 50 of SEQ ID NO:1 or an amino acid sequence having at least 90%, more preferably at least 95%, even more preferably at least 98%, most preferably at least 99% identity with SEQ ID NO:1; - a region of amino acid 221 to 243 of SEQ ID NO:1 or an amino acid sequence having at least 90%, more preferably at least 95%, even more preferably at least 98%, most preferably at least 99% identity with SEQ ID NO:1; and - a region of amino acid 341 to 368 of SEQ ID NO:1 or an amino acid sequence having at least 90%, more preferably at least 95%, even more preferably at least 98%, most preferably at least 99% identity with SEQ ID NO:1.

A described above, the detection of the presence of antibodies against the 5'-nucleotidase according to the invention can be performed my any method known to the skilled person to detect antibodies. However preferably the antibodies in the sample are detected using an antigen or other epitope-carrying compound, specific for said antibodies. Thus, in one embodiment, a 5'-nucleotidase, preferably NT5C1A and/or NT5C1B can be utilized to detect the presence of antibodies, elicited against the 5'-nucleotidase of the subject (e.g. against NT5C1A of the subject), present in a sample. In another embodiment, a fragment (i.e. a stretch of amino acids, adjacently present in the polypeptide from which the fragment is derived or obtained from) of said 5'-nucleotidase, preferably NT5C1A and/or NT5C1B can be utilized to detect the presence of antibodies, elicited against the 5'-nucleotidase of the subject (e.g. against NT5C1A of the subject). Such fragment may in principal be of any length as long as it comprises the epitope to which the antibody in the sample of the subject is directed. Preferably the fragment comprises at least 5, 6 or 7 amino acids adjacently present in the 5'-nucleotidase of the subject (e.g. in NT5C1A).

In a suitable embodiment, the fragment may comprise at least 5, preferably at least 6, more preferably at least 7, such as 8, 9, 10, 11, 12, 13, 14, 15 or more, adjacent amino acids from a region of 5'-nucleotidase selected from the group consisting of: - a region of amino acid 25 to 50 of SEQ ID NO:1 or an amino acid sequence having at least 90%, more preferably at least 95%, even more preferably at least 98%, most preferably at least 99% identity with SEQ ID NO:1; - a region of amino acid 221 to 243 of SEQ ID NO:1 or an amino acid sequence having at least 90%, more preferably at least 95%, even more preferably at least 98%, most preferably at least 99% identity with SEQ ID NO:1; and - a region of amino acid 341 to 368 of SEQ ID NO:1 or an amino acid sequence having at least 90%, more preferably at least 95%, even more preferably at least 98%, most preferably at least 99% identity with SEQ ID NO:1.

A skilled person knows how he can obtain such fragment useful in detecting antibodies in the method according to the invention. For example, he may produce fragments of different length and composition of e.g. NT5C1A and test those for (specific) binding with the antibodies against the 5'-nucleotidase present in a sample obtained from a subject suffering from Inclusion Body Myositis, and comparing binding with samples obtained from e.g. healthy subjects and/or subjects suffering from another idiopathic inflammatory myopathy, including Dermatomyositis and/or Polymyositis. Absence of binding in samples obtained from healthy subjects and/or subjects suffering from another idiopathic inflammatory myopathy, including Dermatomyositis and/or Polymyositis, and presence of binding in a similar sample obtained from a person suffering from Inclusion Body Myositis, and tested positive for the presence of antibodies against 5'-nucleotidase of the subject (e.g. NT5C1A), is indicative of a fragment that can be used in the method according to the invention to detect the said antibodies.

Alternatively the 5'-nucleotidase used to determine interaction with the antibody, or from which the fragment used to determine interaction with the antibody is derived, comprises at least 30, 40, 50, 100, 200, 300, preferably at least 350, most preferably all adjacent amino acids of SEQ ID NO: 1 and/or SEQ ID NO: 2, or of an amino acid sequence having at least 90%, more preferably at least 95%, even more preferably at least 98%, most preferably at least 99% identity with SEQ ID NO:1 and/or SEQ ID NO:2. Indeed, as explained above, the suitability of these 5'-nucleotidases, or amino acid sequences or fragments can easily and straightforward be determined by the skilled person, without undue burden. Suitable 5'-nucleotidase fragments include those encompassing one or more of the major epitope regions recited above.

The 5'-nucleotidase or fragment thereof, used in the detection, may be produced synthetically, or by recombinant means or even be isolated from tissue. It will be understood by the skilled person that the 5'-nucleotidase or fragment thereof, used in the detection of antibodies in a sample obtained from a subject, may further comprise additional amino acid sequences, marker moieties, labels, and the like, and may be, directly or indirectly, bound or affixed to surfaces, carriers, beads, magnetics beads, etc., as long as this does not interfere with the detection of the antibodies according to the invention in a manner that would make the detection unreliable and/or unpredictable.

Preferably the test sample used in all aspects of the current invention is a blood sample. The blood sample may be further treated before use in the methods, uses and kits of the invention.

In another preferred embodiment a method is provided according to the invention further comprising the step of determining the presence of antibodies against at least one antigen selected from the group consisting of Mi-2, Ku, PM/Scl-100, PM/Scl-75, SRP, OJ, EJ, PL-12, PL-7, Ro-52, Jo-1, HisRS, ThrRS, AlaRS, GlyRS, IleRS, AsnRS, TyrRS, PheRS, tRNA^{His}, tRNA^{Ala}, Mi-2alpha, Mi-2beta, SRP54, SRP68, SRP72, Tif1-gamma, MDA5, SAE1, SAE2, serine-tRNA^{Sec}-protein complex, Ro60, La, U1A, U1C, U1-70k, PMS1, PMS2, Ku70, Ku80, eEF1, nuclear RNP and NXP-2 in the test sample.

The antigens above are well-known to the person skilled in the arts relating to autoimmune diseases and specifically idiopathic inflammatory myopathies. The antigens are described in various scientific publications (see for example Mammen AL (2010) Ann N Y Acad Sci 1184: 134-153 and Van Dooren et al. (2011) Autoimmun Highlights 2: 5-20). One drawback of current diagnosis is the relatively low prevalence of antibodies against these antigens in a particular disease. For example, although antibodies against Mi-2 are highly specific for Dermatomyositis, they can be found in 15% to 30% of Dermatomyositis patients. Antibodies against Ku have a prevalence of up to 10% in systemic lupus erythematosus (SLE), but are also detected in 5% to 25% of cases of polymyositis/scleroderma overlap syndrome. Therefore combining the detection a more than one antibody in a test sample obtained from a subject will further improve the diagnosis of the correct condition and lowers the chance of misdiagnosis, which in turn lowers the change of wrong treatment and care.

According to another aspect of the current invention there is provided for the use of 5'-nucleotidase and/or use of a fragment of said 5'-nucleotidase, wherein said fragment is a fragment of at least 5, preferably at least 6, more preferably at least 7 adjacent amino acids derived from said 5'-nucleotidase for detecting antibodies present in a test sample obtained from a subject. In a suitable embodiment, the fragment may comprise at least 5, preferably at least 6, more preferably at least 7, such as 8, 9, 10, 11, 12, 13, 14, 15 or more, adjacent amino acids from a region of 5'-nucleotidase selected from the group consisting of: - a region of amino acid 25 to 50 of SEQ ID NO:1 or an amino acid sequence having at least 90%, more preferably at least 95%, even more preferably at least 98%, most preferably at least 99% identity with SEQ ID NO:1; - a region of amino acid 221 to 243 of SEQ ID NO:1 or an amino acid sequence having at least 90%, more preferably at least 95%, even more preferably at least 98%, most preferably at least 99% identity with SEQ ID NO:1; and - a region of amino acid 341 to 368 of SEQ ID NO:1 or an amino acid sequence having at least 90%, more preferably at least 95%, even more preferably at least 98%, most preferably at least 99% identity with SEQ ID NO:1. In one embodiment the 5'-nucleotidase, or the fragment derived therefrom, used to determine interaction with the antibody is NT5C1A (human 5'-nucleotidase, cytosolic IA) or NT5C1B (human 5'-nucleotidase, cytosolic IB). In a further or other embodiment the 5'-nucleotidase, used to detect the antibodies, or from which the fragment used to determine interaction with the antibody is derived, comprises at least 30, 40, 50, 100, 200, 300, preferably at least 350, most preferably all adjacent amino acids of SEQ ID NO: 1 and/or SEQ ID NO: 2, or of an amino acid sequence having at least 90%, more preferably at least 95%, even more preferably at least 98%, most preferably at least 99% identity with SEQ ID NO:1 and/or SEQ ID NO:2. The use according to the invention is preferably in/for identifying a subject at risk of developing an idiopathic inflammatory myopathy and/or diagnosing a subject suffering from an idiopathic inflammatory myopathy, wherein the idiopathic inflammatory myopathy is selected from the group consisting of Inclusion Body Myositis, Dermatomyositis and/or Polymyositis, most preferably the idiopathic inflammatory myopathy is Inclusion Body Myositis, and/or monitoring progression of Inclusion Body Myositis in a subject. The test sample in the use according to the invention is preferably a blood sample, the subject is preferably a human subject.

In a final aspect of the invention there is provided for a kit. The kit according to the invention comprises means for detecting antibodies against 5'-nucleotidase present in a test sample taken from a subject, preferably wherein the 5'-nucleotidase is NT5C1A (human 5'-nucleotidase, cytosolic IA) or NT5C1B (human 5'-nucleotidase, cytosolic IB) or a 5'-nucleotidase comprising at least 30, 40, 50, 100, 200, 300, preferably at least 350, most preferably all adjacent amino acids of SEQ ID NO: 1 and/or SEQ ID NO: 2, or an amino acid sequence having at least 90%, more preferably at least 95%, even more preferably at least 98%, most preferably at least 99% identity with SEQ ID NO:1 and/or SEQ ID NO:2. In an embodiment, the 5'-nucleotidase may comprise at least 5, preferably at least 6, more preferably at least 7, such as 8, 9, 10, 11, 12, 13, 14, 15 or more, adjacent amino acids from a region selected from the group consisting of: - a region of amino acid 25 to 50 of SEQ ID NO:1 or an amino acid sequence having at least 90%, more preferably at least 95%, even more preferably at least 98%, most preferably at least 99% identity with SEQ ID NO:1; - a region of amino acid 221 to 243 of SEQ ID NO:1 or an amino acid sequence having at least 90%, more preferably at least 95%, even more preferably at least 98%, most preferably at least 99% identity with SEQ ID NO:1; and - a region of amino acid 341 to 368 of SEQ ID NO:1 or an amino acid sequence having at least 90%, more preferably at least 95%, even more preferably at least 98%, most preferably at least 99% identity with SEQ ID NO:1.

The kit according to the invention is preferably for use in identifying a subject at risk of developing an idiopathic inflammatory myopathy and/or diagnosing a subject suffering from an idiopathic inflammatory myopathy, preferably wherein the idiopathic inflammatory myopathy is selected from the group consisting of Inclusion Body Myositis, Dermatomyositis and/or Polymyositis, most preferably the idiopathic inflammatory myopathy is Inclusion Body Myositis, and/or in monitoring progression of Inclusion Body Myositis in a subject.

In one embodiment the kit according to the invention comprises 5'-nucleotidase, preferably wherein the 5'-nucleotidase is NT5C1A (human 5'-nucleotidase, cytosolic IA) or NT5C1B (human 5'-nucleotidase, cytosolic IB) or a 5'-nucleotidase comprising at least 30, 40, 50, 100, 200, 300, preferably at least 350, most preferably all adjacent amino acids of SEQ ID NO: 1 and/or SEQ ID NO: 2, or an amino acid sequence having at least 90%, more preferably at least 95%, even more preferably at least 98%, most preferably at least 99% identity with SEQ ID NO:1 and/or SEQ ID NO:2, or a fragment of said 5'-nucleotidase or said amino acid sequence, wherein said fragment is a fragment of at least 5, preferably at least 6, more preferably at least 7 adjacent amino acids derived from said 5'-nucleotidase, as means for detecting the antibodies against the 5'-nucleotidase. In a suitable embodiment, the fragment may comprise at least 5, preferably at least 6, more preferably at least 7, such as 8, 9, 10, 11, 12, 13, 14, 15 or more, adjacent amino acids from a region of 5'-nucleotidase selected from the group consisting of: - a region of amino acid 25 to 50 of SEQ ID NO:1 or an amino acid sequence having at least 90%, more preferably at least 95%, even more preferably at least 98%, most preferably at least 99% identity with SEQ ID NO:1; - a region of amino acid 221 to 243 of SEQ ID NO:1 or an amino acid sequence having at least 90%, more preferably at least 95%, even more preferably at least 98%, most preferably at least 99% identity with SEQ ID NO:1; and - a region of amino acid 341 to 368 of SEQ ID NO:1 or an amino acid sequence having at least 90%, more preferably at least 95%, even more preferably at least 98%, most preferably at least 99% identity with SEQ ID NO:1. Details with respect to suitable sequences, fragments and the like have already been described above, and are within the knowledge of a skilled person. Said fragment of 5'-nucleotidase may optionally comprise at most 367, such as 365, 360, 350, 300, 250, 200, 150, 100, 90, 80, 70, 60, 50, 45, 40, 35, 30, 25, 20, 19, 18, 17, 16, 15, 14, 13, 12, 11, 10, 9, or 8 amino acids.

The kit according to the invention may be a kit wherein the said 5'-nucleotidase and/or fragment to detect antibodies against 5'-nucleotidase is affixed to a media, preferably selected from the group consisting of a membrane suitable for use in *in vitro* immunoassays, preferably an immunoblot, beads, magnetic beads, carriers, protein-linkers, surfaces. The affixing of the antigen or epitope is known to the skilled person.

The kit may further comprise an assay for the detection of binding of antibodies against 5'-nucleotidase, preferably the assay is selected from the group consisting of a molecular interaction assay, ELISA, immunoblotting, microarrays, immunoprecipitation, immunodiffusion, counterimmunoelectrophoresis, line-blot assay or multiplexed analysis techniques. In the context of the current invention "comprise an assay" may indicate either that the kit comprises all necessary means to perform a particular protocol, that the kit comprises written instructions to perform a particular protocol, and/or comprises only part of the necessary means to perform a particular protocol, but, for example does not comprise the necessary apparatus of specific materials (including the test samples). The above techniques are known to the skilled person and have been reviewed, for example, in Van Dooren et al. (2011) Autoimmun Highlights 2: 5-20.

Van Dooren discloses that a number of multiplex-based assays have been developed during the years, with distinct methodological differences. Multiplex assays are able to screen a single sample of blood or other biological fluid for an array of autoantibody specificities simultaneously. In this way a patient-specific autoantibody profile can be made. One type of a multiplex based assay is the solid surface-based autoantigen microarrays that contain immobilized proteins or other biomolecules in predetermined positions on a solid surface. Interactions between the immobilized antigens and molecules in the serum sample such as (labelled) antibodies can be detected by fluorescence-based procedures. A second type is a so-called addressable-bead autoantigen microarray. Individual antigens of interest are chemically coupled to beads of different colors. Subsequently, sera or other biological fluids can be analyzed in a microtiter well containing a bead mixture. One laser will measure the color of the specific antigen-coupled bead, whereas a second laser determines the presence and quantity of a fluorochrome-coupled secondary antibody bound to the bead. Multiplex-based assays as the described autoantigen microarray techniques have advantages over the conventional techniques in terms of reduced sample volumes, enhanced sensitivity, automation and increased numbers of samples that can be tested.

The line-blot assays, or so-called line immunoassays (LIAs), are based on immunoblotting procedures that spot purified antigens on protein-binding membranes without the need of gel electrophoresis. The laborious purification procedure of native antigens is being replaced by the more reproducible production of highly purified recombinant antigens or synthetic peptides. These developments contribute to the increased sensitivity and specificity of commercially available line-blots. Several LIAs of different manufacturers have recently been clinically validated and the results show that LIAs are becoming a suitable alternative to the more costly and complex techniques sometimes used in diagnostic laboratories.

In another or further embodiment the kit comprises at least one antigen selected from the group consisting of Mi-2, Ku, PM/Scl-100, PM/Scl-75, SRP, OJ, EJ, PL-12, PL-7, Ro-52, Jo-1, HisRS, ThrRS, AlaRS, GlyRS, IleRS, AsnRS, TyrRS, PheRS, tRNA^{His}, tRNA^{Ala}, Mi-2alpha, Mi-2beta, SRP54, SRP68, SRP72, Tif1-gamma, MDA5, SAE1, SAE2, Serine-tRNA^{Sec}-protein complex, Ro60, La, U1A, U1C, U1-70k, PMS1, PMS2, Ku70, Ku80, eEF1, nuclear RNP and NXP-2, for the reason already discussed above. The skilled person will understand that said antigen may also be a fragment, as discussed above for 5'-nucleotidase, derived or obtained from the protein to which the antibody to be detected was elicited, e.g. in the subject from which the sample is obtained. An antigen for use in the kit according to the invention is any (fragment of) a protein that can be used to detect with a certain level of specificity an antibody directed against such protein.

It will be clear that the above description is included to illustrate some embodiments of the invention, and not to limit the scope of protection.

It will be understood by the skilled person that all embodiments and preferences disclosed and described herein can be combined into further and embodiments of the current invention. It will likewise be understood by the skilled person that certain elements or technical features of a first embodiment, aspect or preference may be combined with certain elements or technical features of a second embodiment, aspect or preference, without leaving the scope of the disclosure and invention. For example in case reference is made in a first embodiment to 5'-nucleotidase in a method for detecting progression of Inclusion Body Myositis, and in a second embodiment the 5'-nucleotidase is preferably NT5C1A, the use of NT5C1A in a method for detecting progression of Inclusion Body Myositis is disclosed.

The following non-limiting Examples illustrate the different embodiments of the invention. Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, and Sambrook and Russell (2001) Molecular Cloning: A Laboratory Manual, Third Edition, Cold Spring Harbor Laboratory Press, NY; or Volumes 1 and 2 of Ausubel et al. (1994) Current Protocols in Molecular Biology, Current Protocols, USA. Standard materials.

### EXAMPLES

### Example 1: A biomarker for Inclusion Body Myositis: anti-Mup44, a IBM-specific autoantibody

Here we describe the identification of a skeletal muscle antigen of 44 kDa (Mup44) as an autoantibody target in IBM. Mup44 was recognized by 27% of IBM patient sera. Reactivity with Mup44 in dermatomyositis and polymyositis was 0% and 2%, respectively. These novel IBM-specific autoantibodies and the antigen may be used as biomarker to facilitate the diagnosis of IBM. Autoantibodies to this antigen appear to be highly specific for IBM, showing that it is a novel biomarker for IBM.

### METHODS

### Serum samples

Serum samples from a group of well-characterized inflammatory myopathy patients described by Abdo et al. (Abdo WF, Acta neuropathologica. 2009;118:429-431). (31 IBM, 47 PM and 24 DM patients) were used in this study. For confirmation a separate cohort of serum samples from IBM patients (Badrising UA, Annals of neurology. 2002;51:369-372) as well as serum from healthy individuals obtained from the Sanquin Blood Supply Foundation (Nijmegen, The Netherlands) was analyzed.

### Muscle extract and cell lysates

Human healthy hamstring muscle obtained from reconstructive surgery was frozen in liquid nitrogen and stored at -80 °C. Small pieces of frozen muscle tissue were pulverized with a Microdismembrator (Braun Biotech International, Melsungen, Germany), resuspended in 10 volumes of RIPA buffer (50 mM Tris-CI, pH 7.4, 150 mM NaCl, 1% NP-40, 0.5% DOC, 0.1% SDS, 10 mM DTT, 0.5 mM PMSF and Protease Inhibitor Cocktail (Roche, Mannheim, Germany)), sonicated at 4 °C and centrifuged for 15 minutes at 21,000 g. The supernatant was used for SDS-polyacrylamide gel electrophoresis (SDS-PAGE) followed by western blot analysis.

Human Jurkat T-lymphocyte and mouse C2C12 myoblast cells were cultured according to standard procedures and lysed by sonication in lysis buffer (50 mM Hepes-KOH, pH 7.4, 100 mM KCI, 10 mM MgCl₂, 0.05% NP-40, 0.5 mM PMSF and Protease Inhibitor Cocktail) followed by centrifugation at 21,000 *g.*

### Western blot analysis

Human muscle extracts, Jurkat or C2C12 cell lysates were separated by 12% SDS-PAGE using the full width of the gels, and blotted to nitrocellulose membranes. Membranes were cut into strips of 3-4 mm wide, blocked with 5% milk powder in PBS-0.1% Tween-20 (PBS-T) and incubated with human patient serum, diluted 1:1000 in 5% milk-PBS-T. Bound antibodies were visualized using goat-anti-human-IRDye800 labeled secondary antibody (Rockland, Gilbertsville, USA) and scanned with the Odyssey system (LI-COR Biosciences, Lincoln, USA).

### RESULTS

### Identification of Mup44 in human skeletal muscle extract

To identify autoantibody targets in skeletal muscle tissue, healthy human hamstring extracts were separated by SDS-PAGE, blotted to nitrocellulose and incubated with sera from IBM patients. Several polypeptides were recognized by the antibodies in these patient sera. Although different sera were reactive with distinct muscle antigens, one prominent polypeptide appeared to be commonly detected by several IBM sera. Four out of 9 IBM sera were reactive with this polypeptide with a molecular weight of approximately 44 kDa.

Interestingly, unlike other detected antigens, the 44 kDa polypeptide was skeletal muscle specific. It could not be detected in extracts from cultured cell lines such as human Jurkat and HeLa cells nor in differentiated mouse C2C12 myotubes. It was, however, detected in mouse skeletal muscle extracts and in both healthy as well as IBM-affected skeletal muscle samples. Immunoblotting using adjacent blot strips confirmed that the 44 kDa antigen detected by different IBM patient sera showed exactly the same electrophoretic mobility in SDS-PAGE gels, showing a common antigenic protein, hereafter designated Mup44 (Muscle protein of 44 kDa), is recognized by these sera.

### Association of anti-Mup44 autoantibodies with IBM in myositis sera

To investigate whether anti-Mup44 antibodies are specific for IBM sera or are also found in sera from patients with other inflammatory myopathies, sera from 24 patients with DM and 47 patients with PM were analyzed by immunoblotting as described above. As shown in Table 1,29% of the IBM sera in this cohort contain autoantibodies to Mup44. Although DM and PM sera were reactive with several antigenic proteins in muscle extracts (data not shown), none of the DM and only one PM serum showed reactivity with Mup44 (Table 1).

The relatively high frequency of autoantibodies directed to Mup44 in IBM sera was confirmed in an independent IBM sera cohort, in which 25% of sera were reactive with Mup44. None of 32 sera from healthy individuals did contain anti-Mup44 autoantibodies (Table 1). In total, anti-Mup44 was detected with a sensitivity of 27% and a specificity of 99% in IBM patient sera.

**Table 1. Prevalence of anti-Mup44 autoantibodies.**

| | Number of sera tested | Number of sera recognizing Mup44 | % of sera recognizing Mup44 |
|---|---|---|---|
| IBM^{a} | 31 | 9 | 29 |
| DM^{a} | 24 | 0 | 0 |
| PM^{a} | 47 | 1 | 2 |
| | | | |
| IBM^{b} | 32 | 8 | 25 |
| NHS^{c} | 32 | 0 | 0 |

| | | | |
|---|---|---|---|
| ^{a}Myositis serum cohort (Abdo WF, Acta neuropathologica. 2009;118:429-431); ^{b}IBM serum cohort 2 (Badrising UA, Annals of neurology. 2002;51:369-372) ^{c}Sera from healthy individuals. | | | |

### DISCUSSION

A 44 kDa muscle autoantigen, designated Mup44, which was recognized by 27% of the IBM sera analyzed, whereas it was hardly or not reactive with other myositis and healthy control sera. Anti-Mup44 autoantibodies were detected with a specificity of 99%. The frequency of anti-Mup44 reactivity in IBM sera is relatively high when compared with other autoantibody reactivities in myositis. It is similar to that of the most frequently detected MSA anti-Jo-1, which is targeted by 20%-30% of PM and DM patients. It has been common practice to use cultured cell lines such as HeLa cells for the identification of autoantigens in autoimmune diseases. As a result of the apparent absence of Mup44 expression in such cell lines the patient antibodies to this protein have escaped detection so far. Therefore, anti-Mup44 represents an antibody-based IBM-biomarker which may distinguish IBM from other inflammatory myopathies. This is particularly relevant in view of the difficulty to differentiate PM and IBM based upon the histological analyses of muscle biopsies. We conclude that the presence of anti-Mup44 autoantibodies is a novel biomarker for IBM.

### Example 2: Identification of Mup44 as 5'-nucleotidase IA

To reveal the molecular identity of Mup44 the protein was isolated from healthy skeletal muscle extracts by immunoaffinity chromatography using antibodies isolated from IBM patient sera. SDS-PAGE and staining of the isolated proteins revealed a polypeptide of the expected molecular weight only in the material obtained with antibodies from IBM patients and not with antibodies from controls. The identity of this polypeptide has been determined by mass spectrometric (MS) analysis of tryptic peptides obtained by in-gel digestion.

### METHODS

IgG was isolated from 250 µl serum samples of 2 IBM patients and of a pool of healthy human sera by incubation with 250 µl Protein A-agarose beads (50% slurry; Kem-en-Tec, Denmark). Subsequently, the IgG was covalently coupled to the Protein A-beads by the bifunctional crosslinker dimethyl pimelinediimidate dihydrochloride (Sigma Aldrich) in 0.2 M sodium borate buffer, pH 9.0. Antibody-coupled beads were washed with ethanolamine and PBS and stored at 4 °C until further use.

Human healthy hamstring muscle obtained from reconstructive surgery was frozen in liquid nitrogen and stored at -80 °C. Small pieces of frozen muscle tissue were pulverized with a Microdismembrator (Braun Biotech International, Melsungen, Germany). The resulting material was resuspended in 10 volumes of RIPA buffer (50 mM Tris-HCl, pH 7.4, 150 mM NaCl, 1% Nonidet P-40 (NP-40), 0.5% sodium deoxycholate, 0.1% SDS, 10 mM dithiothreitol, 0.5 mM phenylmethylsulfonyl fluoride (PMSF) and Complete protease inhibitor cocktail (Roche, Mannheim, Germany)), sonicated at 4 °C and centrifuged for 15 minutes at 21,000 g. The supernatant, human muscle lysate, was stored at -80 °C until use in immunoaffinity chromatography.

Human muscle lysate containing 10 mg protein was incubated with 250 µl Protein A-agarose beads (50% slurry) in RIPA buffer to remove endogenous IgG from the muscle lysate. Subsequently, 50 µl antibody-coupled beads were added to the precleared muscle lysate and incubated for 16 hrs at 4 °C. Antibody-coupled beads were washed 5 times with RIPA buffer and bound proteins were released by boiling in SDS-sample buffer (2% SDS, 5% β-mercaptoethanol, 10% glycerol, 0.01% bromophenol bleu, 125 mM Tris-HCl, pH 6.8). Bound proteins were separated by 12% SDS-PAGE and visualized by colloidal Coomassie Brilliant Blue staining.

A gel slice containing the protein band corresponding to the position of Mup44 was excised from the gel, and, after reduction and alkylation, in-gel digested with trypsin. The digested samples were loaded on stagetips for desalting and concentration and eluted in a final volume of 20 µl, of which 5 µl was used for the analysis by means of the nanoLC LTQ FT Ultra MS equipment of the Nijmegen Proteomics Facility (NPF). In brief, the peptide mixture was resolved by reverse phase (RP) nano-HPLC chromatography prior to nano-electrospray ionization. The peptide ions were analyzed by the LTQ FT Ultra MS mass spectrometer.

Peptide and protein identifications were extracted from the data by means of the search program Mascot (http://www.matrixscience.com/search_form_select.html). In this case, the RefSeq33 database with Homo sapiens taxonomy was used with added sequence-tags. Likely contaminants were added to this database (e.g. human keratins, trypsin and LysC). The following modifications were allowed in the search: carbamidomethylation of cysteines (fixed), oxidation of methionine (variable) and acetylation of the N-terminus (variable).

Protein identities were validated by software classifying protein identifications based on the number of uniquely identified peptide sequences, clustering proteins sharing the same set of peptides and validating the proteins based upon the following criteria: proteins with 1 peptide must have a peptide score: >49; proteins with more than 1 peptide must have a peptide score: >29.

### RESULTS

Analysis of the LC-MS/MS results of the excised bands corresponding to Mup44 revealed that Mup44 corresponds to 5'-nucleotidase IA (NP_115915.1). The emPAI scores (Exponentially Modified Protein Abundance Index; http://www.matrixscience.com/help/quantempai help.html) of 5'-nucleotidase IA, which is the highest scoring protein in the LC-MS/MS results listing, were 5.4, 9 and 0.9 for the two IBM and control antibody samples which is in agreement with the intensities of the observed protein bands. Because these high emPAI scores were only obtained for 5'-nucleotidase IA and none of the other identified proteins showed a similar differentiation between IBM and control samples, these results demonstrate that the 44 kDa protein targeted by the IBM patient antibodies is the cytosolic 5'-nucleotidase IA. Importantly, this protein is known to be expressed at relatively high levels in skeletal muscle tissue.

### Example 3: Identification of major epitope regions of 5'-nucleotidase IA targeted by IBM sera

To get more insight into the regions of 5'-nucleotidase IA that are preferentially targeted by IBM sera, microarrays containing a set of overlapping synthetic peptides covering the complete amino acid sequence of 5'-nucleotidase IA (SEQ ID NO:1) were generated and probed with anti-Mup44-positive sera. In total, ninety 15-mer peptides with 11 amino acid overlap were synthesized and spotted in triplicate on the surface of glass slides. These microarrays were purchased from JPT Peptide Technologies (Berlin, Germany). Each slide contained three identical arrays of peptides. The slides were blocked by incubation with MTBST (5% non-fat dried milk in TBS, 0.05% Tween-20) for 1 hour at room temperature. Subsequently the slides were incubated with 300 µl 100-fold diluted patient sera in MTBST for 2 hours at 37 °C in a humid chamber. After washing 5 times with MTBST the arrays were incubated with Alexa Fluor-568-labeled goat-anti-human secondary antibodies (A21090, Molecular Probes) for 1 hour at 30 °C under agitation. After washing 5 times with MTBST and 5 times with water the slides were dried and bound antibodies were visualized by a PerkinElmer ProScanArray microarray scanner. Signals were quantified using Quantity One software (Bio-Rad).

Nine slides were incubated with anti-Mup44 autoantibody-containing IBM sera and two with normal healthy control sera. Three major epitope regions were identified. One of these, located close to the N-terminus, comprises amino acid residues 25 - 50. Some sera also showed weak reactivities with peptides containing parts of the N-terminal 24 amino acids. A second autoreactive region is located close to the C-terminus (a.a. 341 - 368). The third epitope region is more centrally located (a.a. 221 - 243). The control sera were not reactive with any of the 5'-nucleotidase IA peptides on the arrays. These data suggest that 5'-nucleotidase IA contains at least three discontinuous autoepitopes.

## Claims

1. A method for identifying a subject at risk of developing Inclusion Body Myositis, said method comprising the steps of:
a) providing a test sample obtained of said subject; and
b) determining whether autoantibodies against 5'-nucleotidase are present in said sample.

2. A method for distinguishing between subtypes of idiopathic inflammatory myopathy wherein said idiopathic inflammatory myopathy is Dermatomyosis or Polymyosis or Inclusion Body Myositis, in a subject suffering from an idiopathic inflammatory myopathy, said method comprising the steps of:
a) providing a test sample obtained of said subject;
b) determining whether autoantibodies against 5'-nucleotidase are present or absent in said sample.

3. A method for monitoring progression of Inclusion Body Myositis in a subject and/or for determining response to therapy addressing Inclusion Body Myositis in a subject, said method comprising the steps of:
a) providing a first test sample obtained of said subject at a first time point, and a second test sample obtained of said subject at a second time point;
b) determining the level of autoantibodies against 5'-nucleotidase in said first and second samples;
c) comparing the level of antibodies against 5'-nucleotidase in said first sample to said second sample.

4. Method according to any one of the preceding claims, wherein
- the 5'-nucleotidase is NT5C1A (human 5'-nucleotidase, cytosolic IA) or NT5C1B (human 5'-nucleotidase, cytosolic IB) and/or
- the 5'-nucleotidase comprises at least 30 adjacent amino acids of SEQ ID NO:1 and/or SEQ ID NO:2, or of an amino acid sequence having at least 90%identity with SEQ ID NO:1 and/or SEQ ID NO:2.

5. Method according to any one of the preceding claims, wherein the step of determining the presence and/or level of antibodies against 5'-nucleotidase, is performed by determining interaction between said antibodies with 5'-nucleotidase and/or with a fragment of said 5'-nucleotidase, wherein said fragment is a fragment of at least 7 adjacent amino acids derived from said 5'-nucleotidase, wherein the fragment comprises at least 7 adjacent amino acids from a region of 5'-nucleotidase selected from the group consisting of: - a region of amino acid 25 to 50 of SEQ ID NO:1; - a region of amino acid 221 to 243 of SEQ ID NO:1; and - a region of amino acid 341 to 368 of SEQ ID NO:1.

6. Method according to any one of the preceding claims, wherein the 5'-nucleotidase used to determine interaction with the antibody, or from which the fragment used to determine interaction with the antibody is derived, is NT5C1A (human 5'-nucleotidase, cytosolic IA) or NT5C1B (human 5'-nucleotidase, cytosolic IB).

7. Method according to any one of the preceding claims, wherein
- the test sample is a blood sample and/or the subject is a human subject; and/or
- the method comprises the step of determining the presence of antibodies against at least one antigen selected from the group consisting of Mi-2, Ku, PM/Scl-100, PM/Scl-75, SRP, OJ, EJ, PL-12, PL-7, Ro-52, Jo-1, HisRS, ThrRS, AlaRS, GlyRS, IleRS, AsnRS, TyrRS, PheRS, tRNAHis, tRNAAla, Mi-2alpha, Mi-2beta, SRP54, SRP68, SRP72, Tif1-gamma, MDA5, SAE1, SAE2, Serine-tRNASec-protein complex, Ro60, La, U1A, U1C, U1-70k, PMS1, PMS2, Ku70, Ku80, eEF1, nuclear RNP and NXP-2 in the test sample; and/or
- said 5'-nucleotidase is purified recombinant 5'-nucleotidase.

8. A kit comprising means for detecting autoantibodies against 5'-nucleotidase present in a test sample taken from a subject, wherein the kit comprises 5'-nucleotidase or a fragment of said 5'-nucleotidase, wherein said fragment is a fragment of at least 7 adjacent amino acids derived from said 5'-nucleotidase as means for detecting autoantibodies against 5'-nucleotidase, and wherein
the 5'-nucleotidase or a fragment of said 5'-nucleotidase is affixed to a membrane, immunoblot, beads, magnetic beads, carriers, protein-linkers, or surfaces.

9. Kit according to claim 8, wherein the fragment comprises at least 7 adjacent amino acids from a region of 5'-nucleotidase selected from the group consisting of: - a region of amino acid 25 to 50 of SEQ ID NO:1; - a region of amino acid 221 to 243 of SEQ ID NO:1; and - a region of amino acid 341 to 368 of SEQ ID NO:1.

10. Kit according to claim 9, wherein
- the kit further comprises at least one antigen selected from the group consisting of Mi-2, Ku, PM/Scl-100, PM/Scl-75, SRP, OJ, EJ, PL-12, PL-7, Ro-52, Jo-1, HisRS, ThrRS, AlaRS, GlyRS, IleRS, AsnRS, TyrRS, PheRS, tRNAHis, tRNAAla, Mi-2alpha, Mi-2beta, SRP54, SRP68, SRP72, Tif1-gamma, MDA5, SAE1, SAE2, Serine-tRNASec-protein complex, Ro60, La, U1A, U1C, U1-70k, PMS1, PMS2, Ku70, Ku80, eEF1, nuclear RNP and NXP-2; and/or
- said 5'-nucleotidase is purified recombinant 5'-nucleotidase.

11. Use of 5'-nucleotidase and/or use of a fragment of said 5'-nucleotidase, wherein said fragment is a fragment of at least 7 adjacent amino acids derived from said 5'-nucleotidase, for detecting autoantibodies present in a test sample obtained from a subject.

12. Use according to claim 11, wherein
- the 5'-nucleotidase, used to detect the autoantibodies, or from which the fragment used to determine interaction with the autoantibody is derived, comprises at least 30 adjacent amino acids of SEQ ID NO: 1 and/or SEQ ID NO: 2, or of an amino acid sequence having at least 90% identity with SEQ ID NO:1 and/or SEQ ID NO:2, and/or
- the fragment comprises at least 7 adjacent amino acids from a region of 5'-nucleotidase selected from the group consisting of: - a region of amino acid 25 to 50 of SEQ ID NO:1; - a region of amino acid 221 to 243 of SEQ ID NO:1; and - a region of amino acid 341 to 368 of SEQ ID NO:1; and/or
- said 5'-nucleotidase is purified recombinant 5'-nucleotidase.

13. Use according to any one of claims 11-12 in identifying a subject at risk of developing Inclusion Body Myositis and/or diagnosing a subject as suffering from Inclusion Body Myositis, and/or monitoring progression of Inclusion Body Myositis in a subject.

## Patentansprüche

1. Ein Verfahren zum Identifizieren eines Subjekts mit Verdacht auf Entwicklung von Einschlusskörpermyositis, das Verfahren umfasst die Schritte:
a.) Bereitstellen einer Testprobe, die aus dem Subjekt erhalten wurde; und
b.) Bestimmen, ob Autoantikörper gegen 5'-Nukleotidase in der Probe vorhanden sind.

2. Ein Verfahren zum Unterscheiden zwischen Subtypen von idiopathisch entzündlicher Myopathie, wobei die idiopathisch entzündliche Myopathie Dermatomyositis oder Polymyositis oder Einschlusskörpermyositis ist, in einem Subjekt, das unter idiopathisch entzündlicher Myopathie leidet, das Verfahren umfasst die Schritte:
a.) Bereitstellen einer Testprobe, die aus dem Subjekt erhalten wurde;
b.) Bestimmen, ob Autoantikörper gegen 5'-Nukleotidase in der Probe vorhanden sind oder nicht.

3. Ein Verfahren zum Überwachen des Verlaufs von Einschlusskörpermyositis in einem Subjekt und/oder zum Bestimmen der Antwort auf eine Therapie, die auf Einschlusskörpermyositis in einem Subjekt gerichtet ist, das Verfahren umfasst die Schritte:
a.) Bereitstellen einer ersten Testprobe, die aus dem Subjekt zu einem ersten Zeitpunkt erhalten wurde, und einer zweiten Testprobe, die aus dem Subjekt zu einem zweiten Zeitpunkt erhalten wurde;
b.) Bestimmen des Spiegels an Autoantikörpern gegen 5'-Nukleotidase in den ersten und zweiten Proben;
c.) Vergleichen der Spiegel von Antikörpern gegen 5'-Nukleotidase aus der ersten Probe mit der zweiten Probe.

4. Verfahren nach einem der vorangehenden Ansprüche, wobei
- die 5'-Nukleotidase NT5C1A (humane 5'-Nukleotidase, zytosolisch IA) oder NT5C1B (humane 5'-Nukleotidase, zytosolisch IB) ist und/oder
- die 5'-Nukleotidase mindestens 30 nebeneinanderliegende Aminosäuren aus SEQ ID NO:1 und/oder SEQ ID NO:2, oder aus einer Aminosäure-Sequenz, die zu mindestens 90% mit SEQ ID NO:1 und/oder SEQ ID NO:2 identisch ist, umfasst.

5. Verfahren nach einem der vorangehenden Ansprüche, wobei der Schritt des Bestimmens des Vorliegens und/oder des Spiegels der Antikörper gegen 5'-Nukleotidase über das Bestimmen der Wechselwirkung zwischen den Antikörpern mit 5'-Nukleotidase und/oder mit einem Fragment der 5'-Nukleotidase durchgeführt wird, wobei das Fragment ein Fragment aus mindestens 7 nebeneinanderliegenden Aminosäuren abgeleitet von der 5'-Nukleotidase ist, wobei das Fragment mindestens 7 nebeneinanderliegende Aminosäuren aus einer Region der 5'-Nukleotidase umfasst, die ausgewählt sind aus der Gruppe bestehend aus: - einer Region von Aminosäure 25 bis 50 von SEQ ID NO:1; - einer Region von Aminosäure 221 bis 243 von SEQ ID NO:1; und - einer Region von Aminosäure 341 bis 368 von SEQ ID NO:1.

6. Verfahren nach einem der vorangehenden Ansprüche, wobei die 5'-Nukleotidase, die zum Bestimmen der Wechselwirkung mit dem Antikörper verwendet wird, oder aus der das Fragment abgeleitet wird, das zum Bestimmen der Wechselwirkung mit dem Antikörper verwendet wird, NT5C1A (humane 5'-Nukleotidase, zytosolisch IA) oder NT5C1B (humane 5'-Nukleotidase, zytosolisch IB) ist.

7. Verfahren nach einem der vorangehenden Ansprüche, wobei
- die Testprobe eine Blutprobe ist und/oder das Subjekt ein humanes Subjekt ist; und/oder
- das Verfahren den Schritt zum Bestimmen des Vorliegens von Antikörpern gegen mindestens ein Antigen, das ausgewählt ist aus der Gruppe bestehend aus Mi-2, Ku, PM/ScI-100, PM/ScI-75, SRP, OJ, EJ, PL-12, PL-7, Ro-52, Jo-1, HisRS, ThrRS, AlaRS, GlyRS, IleRS, AsnRS, TyrRS, PheRS, tRNAHis, tRNAAla, Mi-2alpha, Mi-2beta, SRP54, SRP68, SRP72, Tif1-gamma, MDA5, SAE1, SAE2, Serine-tRNASec-protein complex, Ro60, La, U1A, U1C, U1-70k, PMS1, PMS2, Ku70, Ku80, eEF1, nukleares RNP und NXP-2, in der Testprobe umfasst; und/oder
- die 5'-Nukleotidase aufgereinigte rekombinante 5'-Nukleotidase ist.

8. Ein Kit umfassend Mittel zum Nachweis von Autoantikörpern gegen 5'-Nukleotidase, die in einer Testprobe entnommen aus einem Subjekt vorliegen, wobei das Kit 5'-Nukleotidase oder ein Fragment der 5'-Nukleotidase umfasst, wobei das Fragment ein Fragment aus mindestens 7 nebeneinanderliegenden Aminosäuren abgeleitet von der 5'-Nukleotidase ist, als Mittel zum Nachweis von Autoantikörpern gegen 5'-Nukleotidase, und wobei
die 5'-Nukleotidase oder ein Fragment der 5'-Nukleotidase an eine Membran, einen Immunblot, Beads, magnetische Beads, Träger, Protein-Linker oder Oberflächen angebracht ist.

9. Kit nach Anspruch 8, wobei das Fragment mindestens 7 nebeneinanderliegende Aminosäuren aus einer Region der 5'-Nukleotidase umfasst, die ausgewählt sind aus der Gruppe bestehend aus: - einer Region von Aminosäure 25 bis 50 von SEQ ID NO:1: - einer Region von Aminosäure 221 bis 243 von SEQ ID NO:1; und - einer Region von Aminosäure 341 bis 368 von SEQ ID NO:1 umfasst.

10. Kit nach Anspruch 9, wobei
- das Kit ferner mindestens ein Antigen ausgewählt aus der Gruppe bestehend aus Mi-2, Ku, PM/ScI-100, PM/Scl-75, SRP, OJ, EJ, PL-12, PL-7, Ro-52, Jo-1, HisRS, ThrRS, AlaRS, GlyRS, IleRS, AsnRS, TyrRS, PheRS, tRNAHis, tRNAAla, Mi-2alpha, Mi-2beta, SRP54, SRP68, SRP72, Tif1-gamma, MDA5, SAE1, SAE2, Serine-tRNASec-protein complex, Ro60, La, U1A, U1C, U1-70k, PMS1, PMS2, Ku70, Ku80, eEF1, nukleares RNP und NXP-2 umfasst; und/oder
- die 5'-Nukleotidase aufgereinigte rekombinante 5'-Nukleotidase ist.

11. Verwendung der 5'-Nukleotidase und/oder Verwendung eines Fragments der 5'-Nukleotidase, wobei das Fragment ein Fragment aus mindestens 7 nebeneinanderliegenden Aminosäuren abgeleitet von der 5'-Nukleotidase zum Nachweis von Autoantikörpern, die in einer aus einem Subjekt erhaltenen Testprobe vorliegen, ist.

12. Verwendung nach Anspruch 11, wobei
- die 5'-Nukleotidase, die zum Nachweis der Autoantikörper verwendet wird, oder aus der das Fragment abgeleitet wird, das zum Bestimmen der Wechselwirkung mit dem Autoantikörper verwendet wird, mindestens 30 nebeneinanderliegende Aminosäuren von SEQ ID NO:1 und/oder SEQ ID NO:2 umfasst, und/oder
- das Fragment mindestens 7 nebeneinanderliegende Aminosäuren aus einer Region der 5'-Nukleotidase umfasst, die ausgewählt sind aus der Gruppe bestehend aus:
- einer Region von Aminosäure 25 bis 50 von SEQ ID NO:1; - einer Region von Aminosäure 221 bis 243 von SEQ ID NO:1; und - einer Region von Aminosäure 431 bis 368 von SEQ ID NO:1 umfasst; und/oder
- die 5'-Nukleotidase aufgereinigte rekombinante 5'-Nukleotidase ist.

13. Verwendung nach einem der Ansprüche 11-12 zum Identifizieren eines Subjekts mit Verdacht auf Entwicklung von Einschlusskörpermyositis und/oder zum Diagnostizieren eines Subjekts, das unter Einschlusskörpermyositis leidet, und/oder zum Überwachen des Verlaufs von Einschlusskörpermyositis in einem Subjekt.

## Revendications

1. Procédé d'identification d'un sujet à risque de développer une myosite à corps d'inclusion, ledit procédé comprenant les étapes de :
a) fourniture d'un échantillon d'essai obtenu à partir dudit sujet ; et
b) détermination de la présence ou non d'auto-anticorps contre la 5'-nucléotidase dans ledit échantillon.

2. Procédé de distinction entre des sous-types de myopathie inflammatoire idiopathique dans lequel ladite myopathie inflammatoire idiopathique est la dermatomyosite ou la polymyosite ou la myosite à corps d'inclusion, chez un sujet souffrant d'une myopathie inflammatoire idiopathique, ledit procédé comprenant les étapes de :
a) fourniture d'un échantillon d'essai obtenu à partir dudit sujet ;
b) détermination de la présence ou de l'absence d'auto-anticorps contre la 5'-nucléotidase dans ledit échantillon.

3. Procédé de surveillance de la progression de la myosite à corps d'inclusion chez un sujet et/ou de détermination de la réponse à une thérapie pour la myosite à corps d'inclusion chez un sujet, ledit procédé comprenant les étapes de :
a) la fourniture d'un premier échantillon d'essai obtenu dudit sujet à un premier temps, et d'un deuxième échantillon d'essai obtenu dudit sujet à un deuxième temps ;
b) la détermination du taux d'auto-anticorps contre la 5'-nucléotidase dans lesdits premier et deuxième échantillons ;
c) la comparaison du taux d'anticorps contre la 5'-nucléotidase dans ledit premier échantillon audit deuxième échantillon.

4. Procédé selon l'une quelconque des revendications précédentes, dans lequel
- la 5'-nucléotidase est NT5C1A (5'-nucléotidase humaine, cytosolique IA) ou NT5C1B (5'-nucléotidase humaine, cytosolique IB) et/ou
- la 5'-nucléotidase comprend au moins 30 acides aminés adjacents de SEQ ID NO: 1 et/ou SEQ ID NO: 2, ou d'une séquence d'acides aminés ayant au moins 90 % d'identité avec SEQ ID NO: 1 et/ou SEQ ID NO: 2.

5. Procédé selon l'une quelconque des revendications précédentes, dans lequel l'étape de détermination de la présence et/ou du taux d'anticorps contre la 5'-nucléotidase, est effectuée par détermination de l'interaction entre lesdits anticorps et la 5'-nucléotidase et/ou avec un fragment de ladite 5'-nucléotidase, dans lequel ledit fragment est un fragment d'au moins 7 acides aminés adjacents dérivé de ladite 5'-nucléotidase, dans lequel le fragment comprend au moins 7 acides aminés adjacents d'une région de 5'-nucléotidase choisie dans le groupe constitué de : - une région des acides aminés 25 à 50 de SEQ ID NO: 1 ; - une région des acides aminés 221 à 243 de SEQ ID NO: 1 ; et - une région des acides aminés 341 à 368 de SEQ ID NO: 1.

6. Procédé selon l'une quelconque des revendications précédentes, dans lequel la 5'-nucléotidase utilisée pour déterminer l'interaction avec l'anticorps, ou à partir duquel le fragment utilisé pour déterminer l'interaction avec l'anticorps est dérivé, est NT5C1A (5'-nucléotidase cytosolique IA humaine) ou NT5C1B (5'-nucléotidase cytosolique IB humaine).

7. Procédé selon l'une quelconque des revendications précédentes, dans lequel
- l'échantillon d'essai est un échantillon de sang et/ou le sujet est un sujet humain ; et/ou
- le procédé comprend l'étape de détermination de la présence d'anticorps contre au moins un antigène choisi dans le groupe constitué de Mi-2, Ku, PM/Scl-100, PM/Scl-75, SRP, OJ, EJ, PL-12, PL-7, Ro-52, Jo-1, HisRS, ThrRS, AlaRS, GlyRS, IleRS, AsnRS, TyrRS, PheRS, ARNtHis, ARNtAla, Mi-2alpha, Mi-2bêta, SRP54, SRP68, SRP72, Tif1-gamma, MDA5, SAE1, SAE2, complexe sérine-ARNtSec-protéine, Ro60, La, U1A, U1C, U1-70k, PMS1, PMS2, Ku70, Ku80, eEF1, RNP nucléaire et NXP-2 dans l'échantillon d'essai ; et/ou
- ladite 5'-nucléotidase est une 5'-nucléotidase recombinante purifiée.

8. Trousse comprenant des moyens pour détecter des auto-anticorps contre la 5'-nucléotidase présente dans un échantillon d'essai prélevé à partir d'un sujet, la trousse comprenant de la 5'-nucléotidase ou un fragment de ladite 5'-nucléotidase, dans laquelle ledit fragment est un fragment d'au moins 7 acides aminés adjacents dérivé de ladite 5'-nucléotidase en tant que moyen pour détecter des auto-anticorps contre la 5'-nucléotidase, et dans laquelle
la 5'-nucléotidase ou un fragment de ladite 5'-nucléotidase est fixée à une membrane, un immunotransfert, des billes, des billes magnétiques, des supports, des lieurs de protéine ou des surfaces.

9. Trousse selon la revendication 8, dans laquelle le fragment comprend au moins 7 acides aminés adjacents d'une région de 5'-nucléotidase choisie dans le groupe constitué de : - une région des acides aminés 25 à 50 de SEQ ID NO: 1 ; - une région des acides aminés 221 à 243 de SEQ ID NO: 1 ; et - une région des acides aminés 341 à 368 de SEQ ID NO: 1.

10. Trousse selon la revendication 9, dans laquelle
- la trousse comprend en outre au moins un antigène choisi dans le groupe constitué de Mi-2, Ku, PM/Scl-100, PM/Scl-75, SRP, OJ, EJ, PL-12, PL-7, Ro-52, Jo-1, HisRS, ThrRS, AlaRS, GlyRS, IleRS, AsnRS, TyrRS, PheRS, ARNtHis, ARNtAla, Mi-2alpha, Mi-2bêta, SRP54, SRP68, SRP72, Tif1-gamma, MDA5, SAE1, SAE2, complexe sérine-ARNtSec-protéine, Ro60, La, U1A, U1C, U1-70k, PMS1, PMS2, Ku70, Ku80, eEF1, RNP nucléaire et NXP-2 ; et/ou
- ladite 5'-nucléotidase est une 5'-nucléotidase recombinante purifiée.

11. Utilisation de 5'-nucléotidase et/ou utilisation d'un fragment de ladite 5'-nucléotidase, dans laquelle ledit fragment est un fragment d'au moins 7 acides aminés adjacents dérivé de ladite 5'-nucléotidase, pour détecter des auto-anticorps présents dans un échantillon d'essai obtenu à partir d'un sujet.

12. Utilisation selon la revendication 11, dans laquelle
- la 5'-nucléotidase, utilisée pour détecter les auto-anticorps, ou à partir de laquelle le fragment utilisé pour déterminer l'interaction avec l'auto-anticorps est dérivé, comprend au moins 30 acides aminés adjacents de SEQ ID NO: 1 et/ou SEQ ID NO: 2, ou d'une séquence d'acides aminés ayant au moins 90 % d'identité avec SEQ ID NO: 1 et/ou SEQ ID NO: 2, et/ou
- le fragment comprend au moins 7 acides aminés adjacents d'une région de 5-nucléotidase choisie dans le groupe constitué de : - une région des acides aminés 25 à 50 de SEQ ID NO: 1 ; - une région des acides aminés 221 à 243 de SEQ ID NO: 1 ; et - une région des acides aminés 341 à 368 de SEQ ID NO: 1 ; et/ou
- ladite 5'-nucléotidase est une 5'-nucléotidase recombinante purifiée.

13. Utilisation selon l'une quelconque des revendications 11 à 12 dans l'identification d'un sujet à risque de développer une myosite à corps d'inclusion et/ou le diagnostic d'un sujet souffrant de myosite à corps d'inclusion, et/ou la surveillance de la progression de la myosite à corps d'inclusion chez un sujet.


### SEQUENCE LISTING

<110> Stichting Katholieke Universiteit
<120> Myositis
<130> P30809NL00
<150> US 61/505,233
   <151> 2011-07-07
<150> NL2007065
   <151> 2011-07-07
<160> 2
<170> PatentIn version 3.5
<210> 1
   <211> 368
   <212> PRT
   <213> Homo sapiens
<400> 1
   Met Glu Pro Gly Gln Pro Arg Glu Pro Gln Glu Pro Arg Glu Pro Gly
   1               5                   10                  15
   Pro Gly Ala Glu Thr Ala Ala Ala Pro Val Trp Glu Glu Ala Lys Ile
               20                  25                  30
   Phe Tyr Asp Asn Leu Ala Pro Lys Lys Lys Pro Lys Ser Pro Lys Pro
           35                  40                  45
   Gln Asn Ala Val Thr Ile Ala Val Ser Ser Arg Ala Leu Phe Arg Met
       50                  55                  60
   Asp Glu Glu Gln Gln Ile Tyr Thr Glu Gln Gly Val Glu Glu Tyr Val
   65                  70                  75                  80
   Arg Tyr Gln Leu Glu His Glu Asn Glu Pro Phe Ser Pro Gly Pro Ala
                   85                  90                  95
   Phe Pro Phe Val Lys Ala Leu Glu Ala Val Asn Arg Arg Leu Arg Glu
               100                 105                 110
   Leu Tyr Pro Asp Ser Glu Asp Val Phe Asp Ile Val Leu Met Thr Asn
           115                 120                 125
   Asn His Ala Gln Val Gly Val Arg Leu Ile Asn Ser Ile Asn His Tyr
       130                 135                 140
   Asp Leu Phe Ile Glu Arg Phe Cys Met Thr Gly Gly Asn Ser Pro Ile
   145                 150                 155                 160
   Cys Tyr Leu Lys Ala Tyr His Thr Asn Leu Tyr Leu Ser Ala Asp Ala
                   165                 170                 175
   Glu Lys Val Arg Glu Ala Ile Asp Glu Gly Ile Ala Ala Ala Thr Ile
               180                 185                 190
   Phe Ser Pro Ser Arg Asp Val Val Val Ser Gln Ser Gln Leu Arg Val
           195                 200                 205
   Ala Phe Asp Gly Asp Ala Val Leu Phe Ser Asp Glu Ser Glu Arg Ile
       210                 215                 220
   Val Lys Ala His Gly Leu Asp Arg Phe Phe Glu His Glu Lys Ala His
   225                 230                 235                 240
   Glu Asn Lys Pro Leu Ala Gln Gly Pro Leu Lys Gly Phe Leu Glu Ala
                   245                 250                 255
   Leu Gly Arg Leu Gln Lys Lys Phe Tyr Ser Lys Gly Leu Arg Leu Glu
               260                 265                 270
   Cys Pro Ile Arg Thr Tyr Leu Val Thr Ala Arg Ser Ala Ala Ser Ser
           275                 280                 285
   Gly Ala Arg Ala Leu Lys Thr Leu Arg Ser Trp Gly Leu Glu Thr Asp
       290                 295                 300
   Glu Ala Leu Phe Leu Ala Gly Ala Pro Lys Gly Pro Leu Leu Glu Lys
   305                 310                 315                 320
   Ile Arg Pro His Ile Phe Phe Asp Asp Gln Met Phe His Val Ala Gly
                   325                 330                 335
   Ala Gln Glu Met Gly Thr Val Ala Ala His Val Pro Tyr Gly Val Ala
               340                 345                 350
   Gln Thr Pro Arg Arg Thr Ala Pro Ala Lys Gln Ala Pro Ser Ala Gln
           355                 360                 365
<210> 2
   <211> 550
   <212> PRT
   <213> Homo sapiens
<400> 2
   Met Ser Gln Thr Ser Leu Lys Gln Lys Lys Asn Glu Pro Gly Met Arg
   1               5                   10                  15
   Ser Ser Lys Glu Ser Leu Glu Ala Glu Lys Arg Lys Glu Ser Asp Lys
               20                  25                  30
   Thr Gly Val Arg Leu Ser Asn Gln Gly Ser Gln Glu Ser Ser Leu Arg
           35                  40                  45
   Lys Thr Asp Ser Arg Gly Tyr Leu Val Arg Ser Gln Trp Ser Arg Ile
       50                  55                  60
   Ser Arg Ser Pro Ser Thr Lys Ala Pro Ser Ile Asp Glu Pro Arg Ser
   65                  70                  75                  80
   Arg Asn Thr Ser Ala Lys Leu Pro Ser Ser Ser Thr Ser Ser Arg Thr
                   85                  90                  95
   Pro Ser Thr Ser Pro Ser Leu His Asp Ser Ser Pro Pro Pro Leu Ser
               100                 105                 110
   Gly Gln Pro Ser Leu Gln Pro Pro Ala Ser Pro Gln Leu Pro Arg Ser
           115                 120                 125
   Leu Asp Ser Arg Pro Pro Thr Pro Pro Glu Pro Asp Pro Gly Ser Arg
       130                 135                 140
   Arg Ser Thr Lys Met Gln Glu Asn Pro Glu Ala Trp Ala Gln Gly Ile
   145                 150                 155                 160
   Val Arg Glu Ile Arg Gln Thr Arg Asp Ser Gln Pro Leu Glu Tyr Ser
                   165                 170                 175
   Arg Thr Ser Pro Thr Glu Trp Lys Ser Ser Ser Gln Arg Arg Gly Ile
               180                 185                 190
   Tyr Pro Ala Ser Thr Gln Leu Asp Arg Asn Ser Leu Ser Glu Gln Gln
           195                 200                 205
   Gln Gln Gln Arg Glu Asp Glu Asp Asp Tyr Glu Ala Ala Tyr Trp Ala
       210                 215                 220
   Ser Met Arg Ser Phe Tyr Glu Lys Asn Pro Ser Cys Ser Arg Pro Trp
   225                 230                 235                 240
   Pro Pro Lys Pro Lys Asn Ala Ile Thr Ile Ala Leu Ser Ser Cys Ala
                   245                 250                 255
   Leu Phe Asn Met Val Asp Gly Arg Lys Ile Tyr Glu Gln Glu Gly Leu
               260                 265                 270
   Glu Lys Tyr Met Glu Tyr Gln Leu Thr Asn Glu Asn Val Ile Leu Thr
           275                 280                 285
   Pro Gly Pro Ala Phe Arg Phe Val Lys Ala Leu Gln Tyr Val Asn Ala
       290                 295                 300
   Arg Leu Arg Asp Leu Tyr Pro Asp Glu Gln Asp Leu Phe Asp Ile Val
   305                 310                 315                 320
   Leu Met Thr Asn Asn His Ala Gln Val Gly Val Arg Leu Ile Asn Ser
                   325                 330                 335
   Val Asn His Tyr Gly Leu Leu Ile Asp Arg Phe Cys Leu Thr Gly Gly
               340                 345                 350
   Lys Asp Pro Ile Gly Tyr Leu Lys Ala Tyr Leu Thr Asn Leu Tyr Ile
           355                 360                 365
   Ala Ala Asp Ser Glu Lys Val Gln Glu Ala Ile Gln Glu Gly Ile Ala
       370                 375                 380
   Ser Ala Thr Met Phe Asp Gly Ala Lys Asp Met Ala Tyr Cys Asp Thr
   385                 390                 395                 400
   Gln Leu Arg Val Ala Phe Asp Gly Asp Ala Val Leu Phe Ser Asp Glu
                   405                 410                 415
   Ser Glu His Phe Thr Lys Glu His Gly Leu Asp Lys Phe Phe Gln Tyr
               420                 425                 430
   Asp Thr Leu Cys Glu Ser Lys Pro Leu Ala Gln Gly Pro Leu Lys Gly
           435                 440                 445
   Phe Leu Glu Asp Leu Gly Arg Leu Gln Lys Lys Phe Tyr Ala Lys Asn
       450                 455                 460
   Glu Arg Leu Leu Cys Pro Ile Arg Thr Tyr Leu Val Thr Ala Arg Ser
   465                 470                 475                 480
   Ala Ala Ser Ser Gly Ala Arg Val Leu Lys Thr Leu Arg Arg Trp Gly
                   485                 490                 495
   Leu Glu Ile Asp Glu Ala Leu Phe Leu Ala Gly Ala Pro Lys Ser Pro
               500                 505                 510
   Ile Leu Val Lys Ile Arg Pro His Ile Phe Phe Asp Asp His Met Phe
           515                 520                 525
   His Ile Glu Gly Ala Gln Arg Leu Gly Ser Ile Ala Ala Tyr Gly Phe
       530                 535                 540
   Asn Lys Lys Phe Ser Ser
   545                 550